(19) Europäisches Patentamt
European Patent Office
Office européen des brevets

(11) Numéro de publication: 0 322 263 B1

(12) FASCICULE DE BREVET EUROPEEN

(45) Date de publication de fascicule du brevet: 30.03.94

(21) Numéro de dépôt: 88402923.2

(22) Date de dépôt: 22.11.88

(51) Int. Cl.5: C07D 498/04, A61K 31/42, A61K 31/435, A61K 31/535, A61K 31/55, C07D 215/14, C07D 215/18, C07D 215/20, C07D 209/12, C07D 223/16, C07D 215/12

# (54) Carbamates tricycliques, leur procédé de préparation et leur application en thérapeutique.

(30) Priorité: 21.12.87 FR 8717842

(43) Date de publication de la demande: 28.06.89 Bulletin 89/26

(45) Mention de la délivrance du brevet: 30.03.94 Bulletin 94/13

(84) Etats contractants désignés: AT BE CH DE ES FR GB GR IT LI LU NL SE

(56) Documents cités:
EP-A- 0 128 120
FR-A- 1 534 278
GB-A- 2 050 357
US-A- 3 391 147

CHEMICAL ABSTRACTS, vol. 88, 1978, page 571, abrégé no. 169920c, Columbus, Ohio, US; M.D. ROZWADOWSKA: "Dihydroquinoline Reissert compounds", & ROCZ. CHEM. 1977, 51(12), 2321-9

(73) Titulaire: SYNTHELABO
22, Avenue Galilée
F-92350 Le Plessis Robinson(FR)

(72) Inventeur: Renaud, Alain
16, Place des Arts
F-92500 Rueil Malmaison(FR)
Inventeur: Schoofs, Alain-René
242, rue de la Croix-Nivert
F-75015 Paris(FR)
Inventeur: Guiraudie, Jean-Marc
95, rue Oberkampf
F-75011 Paris(FR)
Inventeur: Brochet, Denis
55, avenue Jean Jaurès
F-94100 Saint-Maur(FR)

(74) Mandataire: Kedinger, Jean-Paul et al
c/o Cabinet Malemont
42, avenue du Président Wilson
F-75116 Paris (FR)

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**JOURNAL OF THE AMERICAN CHEMICAL SOCIETY**, vol. 92, 1970, pages 2745-2752, Washington, US; F.R. STERMITZ et al.: "Photochemistry of quinoline and some substituted quinoline derivatives"

**CHEMICAL ABSTRACTS**, vol. 78, 1973, page 478, abrégé no. 124521a, Columbus, Ohio, US; V.A. RAO et al.: "Synthesis of 1,2,4,4a,5,6-hexahydro-1,4-oxazino-[3,4-a]quinolines", & INDIAN J. CHEM. 1972, 10(12), 1134-5

**JOURNAL OF THE CHEMICAL SOCIETY, PERKIN II**, 1977, pages 1592-1601, Londres, GB; T.A. CRABB et al.: "Proton magnetic resonance studies of compounds with bridgehead nitrogen. Part 34. Stereochemistry of 8,9,10,11,11a,11b,12,13-octahydro-7aH-quino[1,2-c][1,3]benzoxazines and 7a,8,9,10,10a,10b,11,12-octahydrocyclopent[5,6][1,3]oxazino[3,4-a]quinolines"

**CHEMICAL ABSTRACTS**, vol. 94, 1981, page 636, abrégé no. 156676b, Columbus, Ohio, US; T. SAKATA et al.: "Synthesis of R-(1)-and S-(+)-4-chloro-N-(2-methyl-1-indolinyl)-3-sulfamoylbenzamide", & IYAKUHIN KENYU 1980, 11(3), 388-94

**TETRAHEDRON LETTERS**, vol. 22, no. 51, 1981, pages 5119-5122, Londres, GB; A.I. MEYERS et al.: "Dipole stabilized alpha-amino carbanions. III. Metalation-alkylation of indolines, tetrahydroquinolines and N-methylanilines"

## Description

La présente invention a pour objet de nouveaux carbamates tricycliques, leur procedé de préparation et leur application en thérapeutique, notamment en tant qu'agents antidépresseurs.

Par FR-A-1 534 278 et US-A-3 391 147, on connaît déjà des composés bicycliques du type 1,2 (ou 1,4)-dihydroquinoléine-N-carboxylates d'alcoyle et leur utilisation en tant qu'agents tranquillisants. Par ailleurs, par EP-A-0128120, on connaît des composés bicycliques du type oxazolidinones trisubstituées et leur utilisation en tant qu'agents antidépresseurs.

Les carbamates tricycliques selon l'invention répondent plus précisément à la formule

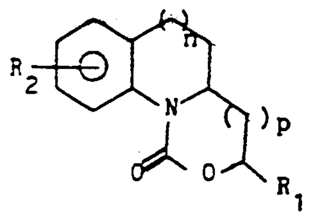

(I)

dans laquelle :

- n = 0, 1 ou 2,
- p = 0 ou 1,
- $R_1$ représente un atome d'hydrogène ; un groupe alkyle en $C_1$-$C_4$ ; un groupe phényle ; un groupe alkényle en $C_2$-$C_3$ ; ou un groupe méthyle substitué par un groupe hydroxy, un groupe alkoxy en $C_1$-$C_4$, un groupe amino ou un groupe N-alkylamino ou N,N-dialkylamino où le reste alkyle est en $C_1$-$C_4$, et
- $R_2$ représente un atome d'hydrogène ; un atome d'halogène ; un groupe alkyle en $C_1$-$C_4$ ; ou un groupe alkoxy en $C_1$-$C_4$.

La présente invention concerne également les sels d'addition d'acide minéral tel que l'acide chlohydrique ou d'acide organique tel qu'un acide carboxylique, des carbamates de formule (I) comportant un groupe salifiable par un tel acide.

Il est à noter que dans ce qui précède et dans ce qui suit, l'expression "alkyle en $C_1$-$C_4$" comprend les groupes à chaîne droite ou ramifiée possédant jusqu'à 4 atomes de carbone, à savoir méthyle, éthyle, n-propyle, i-propyle, n-butyle, i-butyle et tert.-butyle ; l'expression "alkényle en $C_2$-$C_3$" englobe les groupes vinyle, propényle, i-propényle et allyle ; et l'expression "alkoxy en $C_1$-$C_4$" répond à la formule -O-alkyle en $C_1$-$C_4$.

On remarquera également que les composés de formule (I) possèdent un ou deux atomes de carbone asymétriques selon que $R_1$ représente ou non un atome d'hydrogène. Ces composés peuvent donc exister sous un certain nombre de formes stéréoisomères, incluant les énantiomères. La présente invention s'étend donc à chacune de ces formes stéréoisomères, y compris les énantiomères, ainsi qu'à leurs mélanges, y compris les racémates.

Le procédé de préparation des carbamates tricycliques de formule (I) est caractérisé en ce qu'il comprend

(i) la cyclisation, par le carbonate d'éthyle et en présence d'une base (par exemple un alcoolate de métal alcalin tel qu'un alcoolate de sodium) ou par le phosgène, et de préférence dans un solvant organique aprotique comme le toluène, des composés de formule

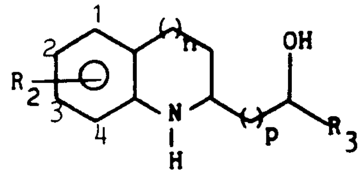

(II)

dans laquelle n, p et $R_2$ ont la même signification que dans la formule (I) définie ci-dessus et $R_3$ représente un atome d'hydrogène, un groupe alkyle en $C_1$-$C_4$, un groupe alkényle en $C_2$-$C_3$ ou un noyau phényle, ou

la cyclisation intramoléculaire en présence d'une base (par exemple un alcoolate de métal alcalin tel qu'un alcoolate de sodium), et de préférence dans un solvant organique aprotique comme le toluène, des composés de formule

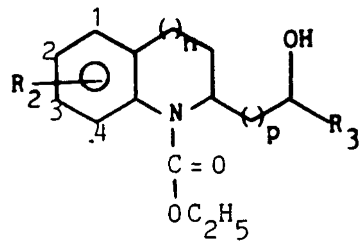

(III)

dans laquelle n, p, $R_2$ et $R_3$ ont la même signification que dans la formule (II) ci-dessus, ce qui conduit aux composés de formule

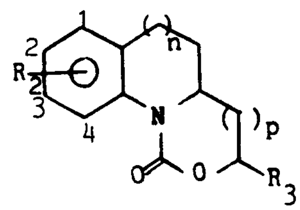

(Ia)

dans laquelle n, p, $R_2$ et $R_3$ ont la même signification que dans la formule (II),
(ii) éventuellement l'ozonolyse des composés de formule (Ia) pour lesquels $R_3$ représente un groupe $CH=CH_2$, cette ozonolyse étant suivie d'une réduction de préférence par un hydrure métallique, ce qui conduit aux composés de formule

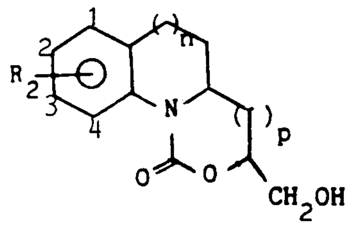

(Ib)

où n, p et $R_2$ ont la même signification que dans la formule (Ia),
(iii) éventuellement l'action d'un agent d'alkylation, de préférence un sulfate de dialkyle en $C_1$-$C_4$ en présence d'un catalyseur de transfert de phase tel que le bromure de tétrabutylammonium, sur le reste $CH_2OH$ des composés de formule (Ib) pour obtenir les composés de formule

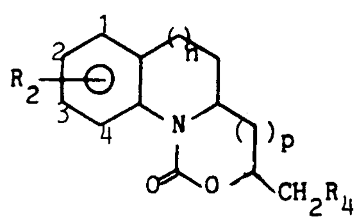

(Ic)

où n, p et $R_2$ ont la même signification que dans la formule (Ib) et $R_4$ représente un groupe alkoxy en $C_1$-$C_4$, et

(iv) éventuellement l'action d'un halogénure de mésyle ou de tosyle sur les composés de formule (Ib), suivie de l'action soit du borohydrure de sodium dans le diméthylsulfoxyde, soit de $NH_3$ ou d'une mono- ou dialkylamine dont le ou les restes alkyle sont en $C_1$-$C_4$, sur les mésylates ou tosylates résultants, ce qui conduit aux composés de formule

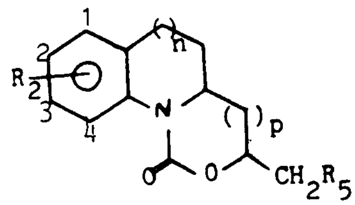

(Id)

où n, p et $R_2$ ont la même signification que dans la formule (Ib) et $R_5$ représente un atome d'hydrogène, un groupe amino ou un groupe alkylamino ou dialkylamino dont le ou les restes alkyle sont en $C_1$-$C_4$.

Les composés de formule (II) sont obtenus (a) par l'action du tert.-butyllithium, de préférence dans un solvant aprotique (THF par exemple), sur les composés de formule

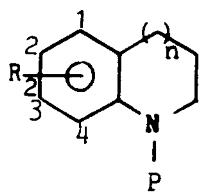

(IV)

dans laquelle n et $R_2$ ont la même signification que dans la formule (I) et P représente un groupe protecteur de l'atome d'azote et apte à activer la position en $\alpha$ de cet atome d'azote, (b) l'action sur le carbanion ainsi formé d'un aldéhyde de formule

$R_3CHO$ (V)

ou d'un époxyde de formule

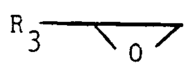

(VI)

$R_3$ ayant dans les formules (V) et (VI) la même signification que dans la formule (II), ce qui conduit aux alcools de formule

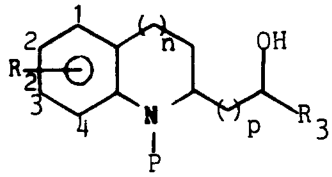

(VII)

où n, p, $R_2$ et $R_3$ ont la même signification que dans la formule (II), puis (c) la déprotection de ces alcools (VII).

Avantageusement, le groupe protecteur P est de formule

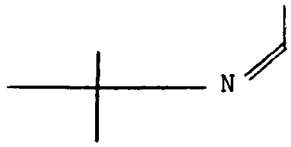

et dans ce cas la déprotection est obtenue par un traitement alcalin en milieu alcoolique, par un traitement à l'hydrure double de lithium et d'aluminium dans l'éther ou par un traitement à l'hydrazine en milieu alcoolique, ce qui génère les composés de formule (II).

Quant aux composés de formule (IV), ils sont préparés de manière connue à partir des indolines, des tétrahydro-1,2,3,4 quinoléines et des tétrahydro-2,3,4,5 benzazépines correspondantes. Ainsi, quand P possède la formule particulière sus-mentionnée, on peut mettre en oeuvre le protocole décrit par A.I. MEYERS, S. HEURING dans Tetrahedron Lett., (1981), 22, 5119.

Les composés de formule (III) pour lesquels $R_3$ représente un groupe alkyle en $C_1$-$C_4$, un groupe alkényle en $C_2$-$C_3$ ou un noyau phényle, sont obtenus par action d'un composé organomagnésien ou organolithien dans lequel le métal est lié à un groupe alkyle en $C_1$-$C_4$, un groupe alkènyle en $C_2$-$C_3$ ou un noyau phényle, sur les composés de formule

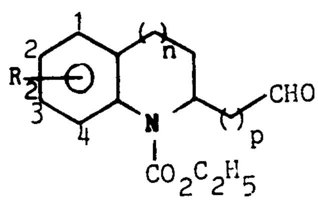

(VIII)

où n, p et $R_2$ ont la même signification que dans la formule (I), suivie d'une hydrolyse.

Les composés de formule (III) pour lesquels $R_3$ représente un atome d'hydrogène sont obtenus par réduction par l'hydrogène en présence d'un catalyseur Pd/C des composés de formule (VIII) ci-dessus définie.

Les composés de formule (VIII) sont obtenus

a) soit par conversion par le nickel de Raney en présence d'hypophosphite de sodium des composés de formule

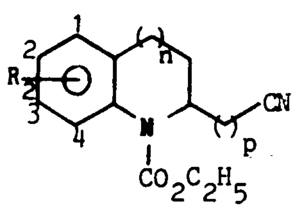

(IX)

où n, p et $R_2$ a la même signification que dans la formule (I),

b) soit par oxydation par le chlorure d'oxalyle en présence de DMSO et de triéthylamine des composés de formule

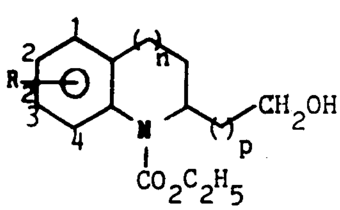

(X)

où n, p et $R_2$ ont la même signification que dans la formule (I).

Les composés de formule (IX) pour lesquels p = 0 et n = 0,1 ou 2 sont obtenus par action du cyanure de triméthylsilyle en présence d'un acide de Lewis comme $AlCl_3$ dans un solvant organique aprotique comme le dichloroéthane sur les composés de formule

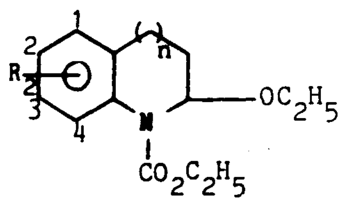

(XI)

où n et $R_2$ ont la même signification que dans la formule (I).

Les composés de formule (IX) pour lesquels p = 0 et n = 1 peuvent en variante être obtenus par hydrogénation en présence d'un catalyseur d'hydrogénation tel que le palladium sur sulfate de baryum ou carbonate de calcium, des composés de formule

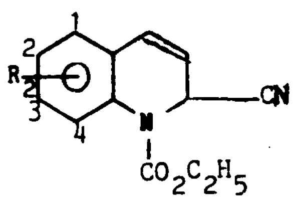

(XII)

où $R_2$ a la même signification que dans la formule (I), ces composés de formule (XII) étant, quant à eux, obtenus par la méthode décrite dans Synthesis (1977), 497.

Les composés de formule (IX) pour lesquels p = 1 et n = 0,1 ou 2 sont obtenus par action d'un halogénure de mésyle ou de tosyle sur les composés de formule (X) pour lesquels p = 0, puis action sur les mésylates ou tosylates ainsi obtenus d'un cyanure de métal alcalin tel que le cyanure de sodium.

Quant aux sels des carbamates de formule (I), ils sont par exemple obtenus par action d'un acide minéral ou organique, en solution dans un solvant approprié, sur une solution dans un solvant approprié des carbamates comportant un groupement salifiable, notamment un groupement amino, alkylamino ou dialkylamino.

Il faut noter enfin que les différentes formes stéréoisomères peuvent être séparées les unes des autres par les méthodes usuelles et notamment par chromatographie liquide en particulier sur colonne de silice.

Les préparations suivantes sont données à titre d'exemples pour illustrer l'invention.

Exemple 1 : (tert-butyliminométhyl)-1 méthoxy-6 (hydroxy-3 propen-1 yl-3)-2 tétrahydro-1,2,3,4 quinoléine [(VII), $R_2$ = 2-$OCH_3$, $R_3$ : $CH=CH_2$, n = 1, p = 0]

La (tert-butyliminométhyl)-1 méthoxy-6 tétrahydro-1,2,3,4 quinoléine (50,5 g, 0,205 mole) est dissoute dans 300 ml de tétrahydrofuranne. La solution est refroidie à - 78° C et le tert-butyllithium 1,7 N dans le pentane (300 ml, 0,51 mole) est ajouté goutte à goutte. Le milieu réactionnel est agité 1 h à une température comprise entre - 15° C et - 20° C avant d'être à nouveau refroidie à - 78° C. L'acroléïne (18 ml, 0,27 mole) en solution dans 30 ml de tétrahydrofuranne est ajoutée à cette température. Après 1 h d'agitation, le milieu est hydrolysé par addition de 300 ml d'eau. Après retour à la température ambiante le milieu est extrait avec trois fois 300 ml de chlorure de méthylène. Les phases organiques sont rassemblées, séchées sur sulfate de sodium et évaporées. Le produit est utilisé brut sans autre purification dans l'exemple 2.

Exemple 2 : méthoxy-6 (hydroxy-3 propen-1 yl-3)-2 tétrahydro-1,2,3,4 quinoléine [(II), $R_2$ = 2-$OCH_3$, $R_3$ : $CH=CH_2$, n = 1, p = 0, code : MD 280 402]

Le produit brut de l'exemple 1 est dissous dans 500 ml de méthanol et une solution d'hydroxyde de potassium 2 N (1160 ml, 10 équivalents) est ajoutée. Après 2 h d'agitation au reflux du solvant, le milieu est

extrait par deux fois 500 ml de chlorure de méthylène. Le produit est extrait des phases organiques par deux fois 500 ml d'une solution d'acide chlorhydrique 3N, les phases aqueuses obtenues sont alcalinisées par une solution concentrée d'hydroxyde de sodium et le produit extrait par trois fois 500 ml de chlorure de méthylène. Après séchage sur sulfate de sodium et évaporation du solvant, on obtient 37 g d'une huile qui est purifiée sur colonne d'alumine en éluant à l'éther isopropylique puis à l'acétate d'éthyle.

$^1H$ RMN ($CDCl_3$) $\delta$ en ppm : 6,5 (3H, singulet large) ; 5,8 (1H, multiplet) ; 5,3 (2H, multiplet) ; 4,0 (1H, multiplet) ; 3,6 (3H, singulet) ; 3,0-3,4 (2H + 1H échangeable, massif) ; 2,6-2,9 (2H, massif) ; 1,6 à 2,0 (2H, massif).

De la même façon, mais à partir des matières premières appropriées, on obtient les autres composés de formule (II) et notamment :

. l'(hydroxy-3 propen-1 yl-3)-2 indoline [$R_2$ = H, $R_3$ : $CH=CH_2$, n = 0, p = 0, code : MD 200 599] sous forme d'huile
  $^1H$ RMN ($CDCl_3$) $\delta$ en ppm : 2,5 (2H) ; 2,9 (2H) ; 3,6 à 4,2 (2H) ; 5 à 6,2 (3H) ; 6,4 à 7,2 (4H).
. l'(hydroxy-3 propen-1 yl-3)-2 tétrahydro-1, 2, 3, 4 quinoléine [$R_2$ = H, $R_3$ : $CH=CH_2$, n = 1, p = 0, code : MD 200 501] sous forme d'huile
  $^1H$ RMN ($CDCl_3$) $\delta$ en ppm : 1,6 à 2,2 (2H) ; 2,7 (2H) ; 3,2 (2H) ; 4 (2H) ; 5 à 5,2 ( 3H) ; 6,4 à 7,2 (4H).

Exemple 3 : éthényl-3 méthoxy-7 tétrahydro-3,3a,4,5 1H-oxazolo [3,4-a] quinoléine-one-1 [(Ia), $R_2$ = 2-$OCH_3$, $R_3$ : $CH=CH_2$, n = 1, p = 0, cis, code : MD 280 403] et [(Ia), $R_2$ = 2-$OCH_3$, $R_3$ : $CH=CH_2$, n = 1, p = 0, trans, code : MD 280 404]

Le produit de l'exemple 2 (10,5 g, 48 mmoles) est dissous dans 100 ml de toluène en présence de carbonate de diéthyle (6,5 g, 55 mmoles). Le milieu est rendu anhydre par distillation azéotropique d'une partie du toluène. La source de chaleur est temporairement retirée et, à 100° C, une solution de méthylate de sodium 4N dans le méthanol (1,2 ml, 4,8 mmoles) est ajoutée. L'éthanol formé par la réaction de cyclisation est distillé sous forme d'azéotrope avec le toluène vers 75-78° C. Lorsque la température du distillat atteint 110° C, la réaction est terminée. Le solvant est évaporé sous vide. Le mélange de diastéréoisomères obtenu est alors soumis à une chromatographie sur silice en employant un système éluant n-heptane/acétate d'éthyle 70/30.

Le produit le moins polaire (MD 280 404) constitué du couple racémique de diastéréoisomères de configuration relative trans est élué en premier : après évaporation des solvants d'élution et recristallisation dans l'éther isopropylique on obtient 2,9 g de ce racémique.

Le produit le plus polaire (MD 280 403) constitué du couple racémique de diastéréoismères de configuration relative cis est élué en second : après évaporation des solvants on obtient 3,8 g (solide blanc) de ce racémique.

Exemple 4 : cyano-2 dihydro-1,2 méthyl-7 quinoléinecarboxylate-1 d'éthyle [(XII), $R_2$ = 3-$CH_3$, code : MD 370 236]

A une solution refroidie à 0° C de 0,6 mole (9,1 g) de méthyl-7 quinoléine dans 110 ml de chlorure de méthylène on ajoute en 5 minutes 0,063 mole (8,5 ml) de cyanure de triméthylsilyle puis en 5 minutes 0,063 mole (6,1 ml) de chloroformiate d'éthyle et 0,44 g de chlorure d'aluminium. Après retour à température ambiante, le milieu réactionnel est versé sur l'eau. La phase organique est lavée à l'eau, séchée sur sulfate de sodium et concentrée. Le produit est purifié par chromatrographie-flash sur silice (éluant : éther isopropylique/éther de pétrole 50/50). On obtient 13,4 g de produit attendu soit un rendement de 87 % (Point de fusion = 87° C).

De la même manière ont été obtenus :

. cyano-2 dihydro-1,2 méthoxy-6 quinoléinecarboxylate-1 d'éthyle : [(XII), $R_2$ = 2-$OCH_3$, code : MD 280 523] ; Point de fusion = 89° C.
. cyano-2 dihydro-1,2 quinoléinecarboxylate-1 d'éthyle : [(XII), $R_2$ = H, code : MD 280 441] ; Point de fusion = 82° C.
. chloro-7 cyano-2 dihydro-1,2 quinoléinecarboxylate-1 d'éthyle [(XII), $R_2$ = 2-Cl, code : MD 370 329] ; huile.

Exemple 5 : cyano-2 tétrahydro-1,2,3,4 quinoléinecarboxylate-1 d'éthyle [(IX), $R_2$ = H , n = 1, p = 0, code : MD 280 442]

Le cyano-2 dihydro-1,2 quinoléinecarboxylate-1 d'éthyle (56,3 g, 0,24 mole) dissous dans 500 ml de méthanol est soumis à une hydrogénolyse sous une pression de 5 kg/cm2 (environ $5.10^5$ Pa) d'hydrogène en présence de palladium à 5 % sur sulfate de barium (5,6 g) à 40° C pendant 2 h. Le catalyseur est éliminé par filtration, le solvant évaporé sous vide et le résidu cristallisé dans l'éther isopropylique. On obtient 48 g d'un solide blanc correspondant au composé attendu et possédant un point de fusion de 84° C.

De la même façon ont été obtenus :

. cyano-2 méthyl-7 tétrahydro-1,2,3,4 quinoléinecarboxylate-1 d'éthyle [(IX), $R_2$ = 3-$CH_3$, n = 1, p = 0, code : MD 370 237] ; Rendement = 80 % ; Point de fusion = 110° C.

. cyano-2 méthoxy-6 tétrahydro-1,2,3,4 quinoléinecarboxylate-1 d'éthyle [(IX), $R_2$ = 2-$OCH_3$, n = 1, p = 0, code : MD 280 524] ; Rendement = 81 % ; Point de fusion = 80° C.

. chloro-7 cyano-2 tétrahydro-1,2,3,4 quinoléinecarboxylate-1 d'éthyle [(IX, $R_2$ = 3-Cl, n = 1, p = 0, code : MD 370 330] ; Rendement = 86 % ; Point de fusion à partir de 65° C.

Exemple 6 : éthoxy-2 tétrahydro-2,3,4,5 1H-benzazépinecarboxylate-1 d'éthyle [(XI), $R_2$ = H, n = 2, code : MD 370 012]

A une suspension de 16,9 g de NaH dans 350 ml de THF, on ajoute une suspension de 0,31 mole (50 g) de tétrahydro-2,3,4,5 1H-benzazépine-one-2 dans 250 ml de THF puis on chauffe le milieu réactionnel 1 h au reflux. Le milieu réactionnel est refroidi à 40° C et on ajoute 0,62 mole (59,57 ml) de chloroformiate d'éthyle en maintenant la température à 40° C et on chauffe à reflux 1 h. Après refroidisement on ajoute 20 ml d'éthanol. Le milieu réactionnel est versé sur la glace. Après extraction à l'éther éthylique on obtient 67 % d'éthoxycarbonyl-1 tétrahydro 2,3,4,5 1H-benzazépine-one-2 (Point de fusion = 74° C). Une solution de 0,075 mole (17,5 g) de cette dernière dans 300 ml d'éthanol est refroidie à - 10° C. On ajoute du bromocrésol, puis simultanément 60 ml d'éthanol chlorhydrique et 0,225 ml de $NaBH_4$ (8,55 g) en maintenant le pH à 5-6 et la température à - 10° C. Puis on laisse remonter la température à 20° C et on ajoute 20 ml d'éthanol chlorhydrdique (3,1 N). Après concentration le résidu est versé sur la glace. La phase aqueuse est extraite à l'éther éthylique. La phase organique est séchée sur sulfate de sodium et concentrée. 17,2 g de produit sous forme d'huile sont obenus :

. IR $\nu$ C = 0 : 1700 $cm^{-1}$

. $^1H$ RMN ($CDCl_3$) $\delta$ ppm : 1 (3H) ; 1,2 (3H) ; 1,6-2 (4H) ; 2,6 (2H) ; 3,6 (2H) ; 4,2 (2H) ; 4-6 (1H) ; 7,1 (4H)

De la même manière on obtient à partir des matières premières correspondantes, l'éthoxy-2 méthoxy-7 tétrahydro-2,3,4,5 1H-benzazépinecarboxylate-1 d'éthyle [(XI), $R_2$ = 2-$OCH_3$, n = 2, code : MD 370 245] :

. IR $\nu$ C = 0 : 1700 $cm^{-1}$

. $^1H$ RMN ($CDCl_3$) $\delta$ ppm : 1 (3H) ; 1,2 (3H) ; 1,6 - 2 (4H) ; 2,6 (2H) ; 3,6 (2H) ; 3,7 (3H) ; 4,2 (2H) ; 4,3 (1H) ; 6,6 - 7,1 (3H).

Exemple 7 : cyano-2 méthoxy-7 tétrahydro-2,3,4,5 1H-benzazépinecarboxylate-1 d'éthyle [(IX), $R_2$ = 2-$OCH_3$, n = 2, p = 0, code : MD 370 246]

A une solution de 0,3 mole (88,5 g) d'éthoxy-2 méthoxy-7 tétrahydro-2,3,4,5 1H-benzazépinecarboxylate-1 d'éthyle [Code : MD 370 245] dans 1 litre de chlorure de méthylène on ajoute à 15° C, 0,392 mole (48,6 ml) de cyanure de triméthylsilyle et 4,02 g de chlorure d'aluminium. On laisse remonter la température à 20° C et après 3 h d'agitation le milieu réactionnel est versé sur l'eau glacée. La phase organique est lavée a l'eau, séchée sur sulfate de sodium et concentrée. L'huile obtenue est utilisée immédiatement pour l'étape suivante.

De la même manière a été obtenu le cyano-2 tétrahydro-2,3,4,5 1H-benzazépinecarboxylate-1 d'éthyle [(IX), $R_2$ = H, n = 2, p = 0,
code : MD 370 013]

IR $\nu$ C ≡ N : 2240$cm^{-1}$, $\nu$ C = 0 : 1700 $cm^{-1}$.

Exemple 8 : carboxaldéhyde-2 tétrahydro-1,2,3,4 quinoléinecarboxylate-1 d'éthyle [(VIII), $R_2$ = H, n = 1, p = 0, code : MD 280 443]

Le composé de l'exemple 5 (46 g, 0,2 mole) est dissous dans un milieu contenant de l'acide acétique (330 ml), de l'eau (330 ml), de la pyridine (660 ml) et de l'hypophosphite de sodium hydraté (92 g). La solution est chauffée à 40° C et du nickel de Raney (37 g) est ajouté par petites quantités, en 5 à 6 heures. Le catalyseur est éliminé par filtration, le filtrat concentré sous vide. Le résidu est repris par de l'eau, le produit extrait par de l'éther. La phase organique est lavée par une solution aqueuse saturée en chlorure d'ammonium puis séchée sur sulfate de sodium. Après évaporation du solvant, une distillation fournit 36,4 g du composé pur attendu.

. point d'ébullition sous 0,05 mm de Hg : 150° C,
. $^1$H RMN ($CDCl_3$) $\delta$ ppm : 1,3 (3H) ; 1,7 à 2,7 (4H) ; 4 à 4,5 (2H) ; 4,5 à 4,9 (1H) ; 6,9 à 7,5 (9H) ; 7,5 à 8,9 (1H) ; 9,6 (1H)
. IR $\nu$ C = 0 : 1745 , 1700 $cm^{-1}$

De la même manière on obtient à partir des matières premières correspondantes, les composés (VIII) suivants :

- carboxaldéhyde-2 chloro-7 tétrahydro-1,2,3,4 quinoléinecarboxylate-1 d'éthyle [$R_2$ = 3-Cl, n = 1, p = 0, code MD 370 331]
  . $_1$H RMN ($CDCl_3$) $\delta$ ppm : 1,3 (3H) ; 2 (2H) ; 2,6 (2H) ; 4,2 (2H) ; 4,7 (1H) ; 6,8 - 7,2 (2H) ; 7,8 (1H) ; 9,5 (1H)
  . IR $\nu$ C = 0 : 1753, 1710 et 1700 $cm^{-1}$
- carboxaldéhyde-2 méthoxy-6 tétrahydro-1,2,3,4 quinoléinecarboxylate-1 d'éthyle [$R_2$ = 2-0$CH_3$, n = 1, p = 0, code : MD 280 525]
  . $^1$H RMN ($CDCl_3$) $\delta$ ppm : 1,2 (3H) ; 1,8 - 2,3 (2H) ; 2,6 (2H) ; 3,7 (3H) ; 4,2 (2H) ; 4,7 (1H) ; 6,5-6,8 (2H) ; 7,6 (1H) ; 9,4 (1H)
  . IR $\nu$ C = 0 : 1740 et 1700 $cm^{-1}$
- carboxaldéhyde-2 tétrahydro-2,3,4,5 1H-benzazépinecarboxylate-1 d'éthyle [$R_2$ = H, n = 2, p = 0, code : MD 370 014]
  . $^1$H RMN ($CDCl_3$) $\delta$ ppm : 1,2 (3H) ; 1,6 - 2 (2H) ; 2,7 (2H) ; 4,2 (2H) ; 4,8 (1H) ; 7 - 7,3 (4H) ; 9,5 (1H)
  . IR $\nu$ C = 0 : 1740 et 1700 $cm^{-1}$
- carboxaldéhyde-2 méthoxy-7 tétrahydro-2,3,4,5 1H-benzazépinecarboxylate-1d'éthyle-1 [$R_2$ = 2-$OCH_3$, n = 2, p = 0, code : MD 370 247]
  . $^1$H RMN ($CDCl_3$) $\delta$ ppm : 1,2 (3H) ; 1,6 - 2,2 (4H) ; 2,7 (2H) ; 3,8 (3H) ; 4,2 (2H) ; 4,3 (1H) ; 6,6 - 6,8 (2H) ; 7,2 (1H) ; 9,3 (1H)
  . IR : $\nu$ C = 0 : 1740 et 1695 $cm^{-1}$

Exemple 9 : éthoxycarbonyl-1 méthoxy-6 tétrahydro-1,2,3,4 quinoléineéthanol-2 [(X), $R_2$ = 2-$OCH_3$, n = 1, p = 1, code : MD 370 315]

A une solution de 0,084 mole (19,4 g) [d'après JOC, 16, p. 895 (1951)] d'ester éthylique de l'acide méthoxy-6 quinoléine-2 acétique dans 200 ml de diméthoxyéthane, on ajoute peut à petit à 40° C 0,084 mole (1,8 g) de borohydrure de lithium. On chauffe 5 minutes à reflux le milieu réactionnel. Après refroidissement, le milieu réactionnel est versé sur l'eau et le produit est extrait par le chlorure de méthylène. Après purification par chromatographie sur colonne de silice (éluant : heptane/acétate d'éthyle 70/30), le produit est recristallisé dans l'éther isopropylique (Rendement = 65 % ; Point de fusion : 88° C).
A une solution de 0,054 mole (11g) du produit précédent dans 150 ml de méthanol avec quelques gouttes d'éthanol chlorhydrique (3 N), on ajoute 1,1 g d'oxyde de platine et on y fait barboter un courant d'hydrogène pendant 12 h. Après filtration, la phase organique est concentrée. Le résidu est repris dans le chlorure de méthylène. La phase organique est lavée à l'eau bicarbonatée puis à l'eau, séchée sur sulfate de sodium et concentrée. Après purification par chromatographie (éluant : heptane/acétate d'éthyle 40/60) on obtient 4,7 g de produit sous forme d'huile. 0,0197 mole de cette huile (4,1 g) est dissoute dans 100 ml de chloroforme auquel on ajoute 0,0394 mole (5,4 g) de carbonate de potassium et 0,0237 mole (2,3 ml) de chloroformiate d'éthyle. Le milieu réactionnel est chauffé au reflux 2 h. Après refroidissement, filtration et concentration, le résidu est purifié par chromatographie sur colonne de silice (éluant : heptane/acétate d'éthyle 60/40). Le produit attendu obtenu est isolé avec un rendement de 80 % sous forme d'huile.

. $^1$H RMN ($CDCl_3$) $\delta$ ppm : 1,2 (3H) ; 1,4 à 1,8 (2H) ; 2 à 2,8 (4H) ; 3 à 3,7 (3H) ; 3,7 (3H) ; 4,2 (2H) ; 4,7 (1H) ; 6,5 à 6,8 (9H) ; 7,2 (1H)

. IR : ν OH : 3450 $cm^{-1}$

Exemple 10 : éthoxycarbonyl-1 méthoxy-6 tétrahydro-1,2,3,4 quinoléine-2 acétaldéhyde [(VIII), $R_2$ = 2-$OCH_3$, n = 1, p = 1, code : MD 370 316]

A une solution de O,016 mole (2g) de chlorure d'oxalyle dans 26 ml de chlorure de méthylène on ajoute 0,0292 mole de DMSO dans 16 ml de chlorure de méthylène refroidie à - 60° C puis on ajoute 0,0146 mole (4,1 g) du composé obtenu à l'exemple 9, en solution dans 16 ml de chlorure de méthylène. Puis après 15 minutes on ajoute 0,073 mole de triéthylamine en solution dans 16 ml de chlorure de méthylène. On laise remonter la température à + 20° C. Le milieu réactionnel est concentré à sec, le résidu est repris dans un mélange eau - chlorure de méthylène. La phase organique est lavée à l'eau, séchée sur sulfate de sodium et concentrée. Le produit attendu obtenu est isolé sous forme d'huile après chromatographie sur colonne de silice (éluant : heptane/acétate d'éthyle 90/10).

. Rendement : 69 %
. IR : ν C = 0 : 1770 $cm^{-1}$ et 1745 $cm^{-1}$
. $^1H$ RMN ($CDCl_3$) δ ppm : 1,2 (3H) ; 1,4 à 1,7 (2H) ; 2 à 2,8 (4H) ; 3,7 (3H) ; 4,2 (2H) ; 5 (1H) ; 6,5 à 6,8 (2H) ; 7,3 (1H) ; 9,5 (1H)

Exemple 11 : éthényl-3 tétrahydro-3,3a,4,5 1H-oxazolo [3,4-a] quinoléine-one-1 [(Ia), n = 1, p = 0, $R_2$ = H, $R_3$ = $CH=CH_2$, cis, code : MD 200 502] et [(Ia), n = 1, p = 0, $R_2$ = H, $R_3$ = $CH=CH_2$, trans, code : MD 200 503]

Le bromure de vinyl magnésien (0,165 mole) préparé à partir de bromure de vinyle (11,6 ml, 0,165 mole) et de magnésium (3,86 g, 0,165 mole) dans 80 ml de tétrahydrofuranne est ajouté goutte à goutte à une solution du composé de code MD 280 443 (préparé à l'exemple 8) dans 350 ml de tétrahydrofuranne, maintenue à une température comprise entre - 20° C et - 40° C. L'agitation est maintenue ½ h après la fin de l'addition, puis une solution aqueuse saturée en chlorure d'ammonium est introduite. Le milieu réactionnel est extrait à l'éther éthylique, la phase organique lavée par une solution aqueuse saturée en chlorure de sodium, séchée sur sulfate de sodium, puis évaporée sous vide. Le résidu est repris par 500 ml de toluène et traité comme dans l'exemple 3 par du méthylate de sodium pour achever la cyclisation. Le toluène est ensuite évaporé, le milieu repris par le chlorure de méthylène, lavé à l'eau, séché sur sulfate de sodium puis évaporé. Le mélange des deux couples de diastéréoisomères obtenu (32,6 g) est alors séparé par chromatographie liquide sur silice.

Le produit le moins polaire (code : MD 200 503) est constitué par le couple racémique de diastéréoisomères de configuration relative trans.
Le produit le plus polaire (code : MD 200 502) est constitué par le couple racémique de diastéréoisomères de configuration relative cis.

Exemple 12 : trans hydroxyméthyl-3 tétrahydro-3,3a,4,5 1H-oxazolo [3,4-a] quinoléine-one-1 [(Ib), n = 1, p = 0, $R_2$ = H, code : MD 200 505]

Le produit trans de code MD 200 503 (2,5 g, 11,6 mmoles) est dissous dans un mélange de méthanol (45 ml) et de chlorure de méthylène (35 ml). On fait passer à travers la solution refroidie à -60° C un courant d'ozone pendant 1 h (débit gazeux de 0,8 l/mn contenant 0,6 M d'ozone). On laisse ensuite remonter la température du milieu réactionnel entre - 20° C et - 30° C avant d'ajouter le borohydrure de sodium (7,4 g 19,7 mmoles). Après 30 mn d'agitation à cette température, on ajoute du diméthylsulfure (11 ml, 15 mmoles). L'agitation est maintenue 3 h supplémentaires en laissant le milieu revenir à température ambiante. Le milieu réactionnel est versé dans une solution d'acide chlorhydrique 1N, le produit est extrait par le chlorure de méthylène, la phase organique séchée sur sulfate de sodium et évaporée sous vide. Après purification sur une colonne de silice (système éluant n-heptane/acétate d'éthyle 20/80) on obtient 2,3 g du produit pur attendu.

Exemple 13 : cis méthoxyméthyl-3 tétrahydro-3,3a,4,5 1H-oxazolo [3,4-a] quinoléine-one-1 [(Ic), $R_4$ = $OCH_3$, $R_2$ = H, n = 1, p = 0, code : MD 200 682]

Le composé cis hydroxyméthyl-3 tétrahydro-3,3a,4,5 1H-oxazolo [3,4-a] quinoléine-one-1 [(Ib) ; code : MD 200 504] (5,6 g, 25,5 mmoles) est mis en suspension dans 100 ml de toluéne. A cette suspension sont ajoutés du bromure de tétrabutylammonium (820 mg, 2,5 mmoles), du diméthylsulfate (7,2 ml, 76 mmoles),

puis une solution d'hydroxyde de sodium à 50 % (127 mmoles). Le milieu réactionnel est agité pendant une nuit à température ambiante, puis additionné d'eau (100 ml). La phase aqueuse est décantée puis extraite au toluène. Les phases organiques sont rassemblées, séchées sur sulfate de sodium, puis évaporées sous vide. Le résidu est cristallisé dans un mélange éther éthylique - éther de pétrole. Une flash chromatographie sur silice (éluant : n-heptane/acétate d'éthyle 50/50) fournit 4,3 g du produit pur attendu.

Exemple 14 : méthanesulfonate de cis méthoxy-7 tétrahydro-3,3a,4,5 1H-oxazolo [3,4-a] quinoléine-one-1 yl-3 méthanol [code : MD 370 046]

A une suspension refroidie à 0° C de 0,0224 mole (5,6 g) de cis hydroxyméthyl-3 méthoxy-7 tétrahydro-3,3a,4,5 1H- oxazino [3,4a] quinoléine-one-1 (code : MD 280 386) dans 130 ml de chlorure de méthylène on ajoute 0,026 mole (3,6 ml) de triéthylamine et 0,026 mole (2 ml) de chlorure de mésyle. Puis le milieu réactionnel est laissé 1/2 heure à la température ambiante et versé sur l'eau. La phase organique est séchée sur sulfate de sodium et concentrée. Le produit attendu obtenu est isolé avec un rendement de 97 % (Point de fusion = 164° C).

De la même manière on obtient à partir des matières premières correspondantes, le méthanesulfonate du trans méthoxy-7 tétrahydro-3,3a, 4,5 1H-oxazolo [3,4a] quinoléine-one-1 yl-3 méthanol [code : MD 370 074]

. Rendement : 95 %
. Point de fusion : 177° C

Exemple 15 : trans méthyl-3 tétrahydro-3,3a,4,5 1H-oxazolo [3,4a] quinoléine-one-1 [(Id), $R_2$ = H, n = 1, p = 0, $R_5$ = H, code : MD 280430]

A une solution de O,9.$10^{-3}$ mole (0,2 g) du composé préparé à l'exemple 12, dans 4 ml de pyridine, on ajoute 1,27.$10^{-3}$ mole de chlorure de tosyle (0,24 g) à 0° C. Après 4 h de contact, le milieu réactionnel est versé sur l'eau glacée et le tosylate obtenu est filtré (Point de fusion = 186° C). A 5 ml de DMSO on ajoute 1,07.$10^{-3}$ mole de borohydrure de sodium et 0,5.$10^{-3}$ mole (0,2 g) du tosylate précédent et on chauffe à 90° C pendant 40 minutes. Le milieu réactionnel est versé sur l'eau et extrait à l'éther éthylique. La phase organique est séchée sur sulfate de sodium et concentrée. Le produit attendu obtenu est isolé avec un rendement dfe 72 %.

Exemple 16 : cis aminométhyl-3 méthoxy-7 tétrahydro-3,3a,4,5 1H-oxazolo [3,4a] quinoléine-one-2 [(Id), $R_2$ = 2-$OCH_3$, n = 1, p = 0, $R_5$ = $NH_2$, code : MD 370 024]

Dans un réacteur résistant à la pression, on place une solution de 0,024 mole (6g) de méthanesulfonate de cis méthoxy-7 tétrahydro-3,3a,4,5 1H-oxazolo [3,4a] quinoléine-one-1 yl-3 méthanol [code : MD 370 046] dans 40 ml de méthanol et 20 ml d'éthanol et 60 ml d'ammoniac liquide. On chauffe à 80° C pendant 12 h. Le milieu réactionnel est concentré et le résidu est repris dans le chlorure de méthylène. La phase organique est lavée à l'eau et à l'ammoniaque, séchée et concentrée. Le produit attendu obtenu est isolé sous forme de chlorhydrate avec un rendement de 20 %.

En utilisant les modes opératoires ci-dessus, mais à partir des réactifs appropriés, on obtient les autres composés selon l'invention. Les caractéristiques physiques d'un certain nombre de composés (I) sont rassemblées dans le Tableau I ci-après ; il est à noter que les composés répertoriés dans ce Tableau se présentent sous la forme de couple racémique de diastéréoisomères de configuration relative cis ou trans.

TABLEAU I

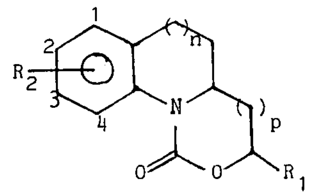

(I)

| Code | n | p | $R_2$ | $R_1$ | Configuration | Base ou sel | Formule brute | F°C | R.M.N. | | | I.R.$\nu$C = 0 $cm^{-1}$ |
|---|---|---|---|---|---|---|---|---|---|---|---|---|
| | | | | | | | | | Noyau | Solvant | δ p.p.m. | |
| MD 200602 | 0 | 0 | H | $CH_2OH$ | cis | base | $C_{11}H_{11}NO_2$ | 128 | $^1H$ | $DMSOd_6$ | 2,8 à 3,4 (2H) ; 3,7 (2H); 4,7 à 5,2 (3H) ; 6,8 à 7,4 (4H) | 1730 |
| MD 200718 | 0 | 0 | H | $CH_2OH$ | trans | base | $C_{11}H_{11}NO_3$ | 113 | $^1H$ | $DMSOd_6$ | 3 à 3,4 (2H) ; 3,7 (2H) ; 4,4 à 4,8 (2H) ; 5,2 (1H) ; 7 à 7,4 (4H) | 1740 |
| MD 200600 | 0 | 0 | H | $CH=CH_2$ | cis | base | $C_{12}H_{11}NO_2$ | 74 | $^1H$ | $CDCl_3$ | 2,6 à 3,4 (2H) ; 4,7 à 6,2 (5H) ; 6,8 à 7,4 (4H) | 1750 |
| MD 200601 | 0 | 0 | H | $CH=CH_2$ | trans | base | $C_{12}H_{11}NO_2$ | - | $^1H$ | $CDCl_3$ | 2,8 à 3,4 (2H) ; 4,2 à 5 (2H) ; 5,2 à 6,4 (3H) ; 6,8 à 7,4 (4H) | 1760 |
| MD 200504 | 1 | 0 | H | $CH_2OH$ | cis | base | $C_{12}H_{13}NO_3$ | 131 | $^1H$ | $DMSOd_6$ | 1,8 à 2,2 (2H); 2,8 (2H) ; 3,6 (2H); 4 à 4,8 (2H) ; 5 (1H) ; 6,8 à 7,3 (3H) ; 8,1 (1H) | 1700 |

TABLEAU I

(suite)

| Code | n | p | $R_2$ | $R_1$ | Configuration | Base ou sel | Formule brute | F°C | R.M.N. Noyau | R.M.N. Solvant | R.M.N. δ p.p.m. | I.R.νC = O $cm^{-1}$ |
|---|---|---|---|---|---|---|---|---|---|---|---|---|
| MD 200505 | 1 | 0 | H | $CH_2OH$ | trans | base | $C_{12}H_{13}NO_3$ | 138 | $^1H$ | $CDCl_3$ | 1,5 à 2,2 (2H) ; 2,8 (2H) ; 3,7 (2H) ; 4 (1H); 4,3 (1H); 5,2 (1H) ; 6,8 à 7,3 (3H) ; 8,1 (1H) | 1720 |
| MD 200502 | 1 | 0 | H | $CH=CH_2$ | cis | base | $C_{13}H_{13}NO_2$ | 91 | $^1H$ | $CDCl_3$ | 1,6 à 2,2 (2H); 2,8 (2H) ; 3,9 à 4,3 (1H); 4,8 à 6,2 (4H) ; 6,8 à 7,4 (3H) ; 8,2 (1H) | 1740 |
| MD 200503 | 1 | 0 | H | $CH=CH_2$ | trans | base | $C_{13}H_{13}NO_2$ | 121 | $^1H$ | $CDCl_3$ | 1,6 à 2,4 (2H); 2,9 (2H) ; 3,8 (1H) ; 4,6 (1H) ; 5,3 à 6,3 (3H); 6,8 à 7,4 (3H) ; 8,3 (1H) | 1760 |
| MD 200682 | 1 | 0 | H | $CH_2-OCH_3$ | cis | base | $C_{13}H_{15}NO_3$ | 113 | $^1H$ | $CDCl_3$ | 1,6 à 2,2 (2H); 2,8 (2H) ; 3,4 (3H) ; 3,6 (2H) ; 4,4 à 4,8 (2H); 6,8 à 7,2 (3H) ; 8,1 (1H) | 1740 |
| MD 200683 | 1 | 0 | H | $CH_2-OCH_3$ | trans | base | $C_{13}H_{15}NO_3$ | 69 | $^1H$ | $CDCl_3$ | 1,5 à 2,4 (2H) ; 2,8 (2H) ; 3,4 (3H) ; 3,6 (2H) ; 3,6 à 4,4 (2H) ; 6,8 à 7,2 (3H) ; 8,1 (1H) | 1750 |

TABLEAU I

(suite)

| Code | n | p | $R_2$ | $R_1$ | Confi-guration | Base ou sel | Formule brute | F°C | R.M.N. | | | I.R.νC = 0 $cm^{-1}$ |
|---|---|---|---|---|---|---|---|---|---|---|---|---|
| | | | | | | | | | Noyau | Solvant | δ p.p.m. | |
| MD 280389 | 1 | 0 | H | $C_6H_5$ | cis | base | $C_{17}H_{15}NO_2$ | 123 | $^1H$ | $CDCl_3$ | 1 à 1,8 (2H) ; 2,8 (2H) ; 4,2 à 4,6 (1H) ; 5,7 (1H) ; 7 à 7,4 (8H) ; 8,2 (1H) | 1740 |
| MD 280390 | 1 | 0 | H | $C_6H_5$ | trans | base | $C_{17}H_{15}NO_2$ | 135 | $^1H$ | $CDCl_3$ | 1,6 à 2,4 (2H) ; 2,9 (2H) ; 3,9 (1H); 5,1 (1H) ; 7 à 7,4 (3H) ; 8,3 (1H) | 1750 |
| MD 280526 | 1 | 0 | H | H | _ | base | $C_{11}H_{11}NO_2$ | 95 | $^1H$ | $CDCl_3$ | 1,1 (3H); 1,5 à 2,2 (4H) ; 2,8 (2H); 3,9 à 4,7 (2H) ; 8,1 (1H) | 1750 |
| MD 280429 | 1 | 0 | H | $CH_3$ | cis | base | $C_{12}H_{13}NO_2$ | 114 | $^1H$ | $CDCl_3$ | 1,4 (3H) ; 1,6 à 2,2 (2H) ; 2,9 (2H) ; 4,1 (1H) ; 4,8 (1H) ; 6,8 à 7,4 (3H) ; 8,2 (1H) | 1730 |
| MD 280430 | 1 | 0 | H | $CH_3$ | trans | base | $C_{12}H_{13}NO_2$ | 128 | $^1H$ | $CDCl_3$ | 1,4 (3H) ; 1,5 à 2,3 (2H) ; 2,8 (2H); 3,7 (1H); 4,4 (1H); 6,8 à 7,3 (3H) ; 8,1 (1H) | 1740 |
| MD 280460 | 1 | 0 | H | $C_2H_5$ | cis | base | $C_{13}H_{15}NO_2$ | 114 | $^1H$ | $CDCl_3$ | 1,1 (3H); 1,6 à 2,3 (4H) ; 2,9 (2H); 3,6 à 4,4 (2H) ; 6,8 à 7,4 (3H) ; 8,2 (1H) | 1750 |

TABLEAU I

(suite)

| Code | n | p | $R_2$ | $R_1$ | Configuration | Base ou sel | Formule brute | F°C | R.M.N. Noyau | R.M.N. Solvant | R.M.N. δ p.p.m. | I.R. νC = 0 $cm^{-1}$ |
|---|---|---|---|---|---|---|---|---|---|---|---|---|
| MD 280459 | 1 | 0 | H | $C_2H_5$ | trans | base | $C_{13}H_{15}NO_2$ | 96 | $^1H$ | $CDCl_3$ | 1,1 (3H); 1,6 à 2,3 (4H) ; 2,9 (2H); 3,6 à 4,4 (2H) ; 6,8 à 7,4 (3H) ; 8,2 (1H) | 1750 |
| MD 370030 | 1 | 0 | H | $CH_2N-(CH_3)_2$ | cis | sel HCl | $C_{14}H_{19}ClN_2O_2$ | >250 | $^1H$ | $DMSOd_6$ | 1,6 à 2,4 (2H); 2,8 (8H) ; 3,6 (2H) ; 4,4 (1H); 5,3(1H) 7 à 7,4 (3H) ; 8 (1H) | 1745 |
| MD 370031 | 1 | 0 | H | $CH_2N-(CH_3)_2$ | trans | sel HCl | $C_{14}H_{19}ClN_2O_2$ | >250 | $^1H$ | $DMSOd_6$ | 1,6 à 2,4 (2H); 2,9 (8H) ; 3,6 (2H); 4 (1H); 5 (1H); 7 à 7,4 (3H) ; 8,2 (1H) | 1760 |
| MD 370032 | 1 | 0 | 3-$CH_3$ | $CH_2OH$ | cis | base | $C_{13}H_{15}NO_3$ | 160 | $^1H$ | $CDCl_3$ | 2,1 (2H); 2,3 (3H); 2,9 (2H); 3,8 (3H); 4,3 (1H); 4,7 (1H); 6,8 à 7,2 (2H) ; 8 (1H) | 1715 |
| MD 370033 | 1 | 0 | 3-$CH_3$ | $CH_2OH$ | trans | base | $C_{13}H_{15}NO_3$ | 139 | $^1H$ | $CDCl_3$ | 1,8 à 2,4 (2H); 2,3 (3H) ; 2,9 (2H); 3,9 (3H); 4,2 (1H); 4,6 (1H); 6,7 à 7,1 (2H) ; 8 (1H) | 1715 |
| MD 370336 | 1 | 0 | 3-Cl | $CH_2OH$ | cis | base | $C_{12}H_{12}ClNO_3$ | 172 | $^1H$ | $DMSOd_6$ | 1,6 à 2,4 (2H); 2,8 (2H) ; 3,7 (2H); 4 à 4,8 (2H) ; 5,2 (1H); 6,9 à 7,3 (2H) ; 8,2 (1H) | 1720 |

<u>T A B L E A U I</u>

(suite)

| Code | n | p | $R_2$ | $R_1$ | Configuration | Base ou sel | Formule brute | F°C | R.M.N. Noyau | R.M.N. Solvant | R.M.N. δ p.p.m. | I.R.υC = O $cm^{-1}$ |
|---|---|---|---|---|---|---|---|---|---|---|---|---|
| D 370335 | 1 | 0 | 3-Cl | $CH_2OH$ | trans | base | $C_{12}H_{12}ClNO_3$ | 152 | $^1H$ | $DMSOd_6$ | 1,5 à 2,4 (2H); 2,8 (2H) ; 3,6 à 4,5 (4H) ; 5,2 (1H); 6,9 à 7,3 (2H) ; 8,1 (1H) | 1730 |
| D 370038 | 1 | 1 | 2-$OCH_3$ | $CH_2OH$ | le plus polaire | base | $C_{14}H_{17}NO_4$ | 144 | $^1H$ | $CDCl_3$ | 1,7 à 2,6 (5H); 2,9 (2H) ; 3,8 (6H), 4,5 (1H); 6,6 à 6,8 (2H) ; 7,8 (1H) | 1720 |
| MD 370039 | 1 | 1 | 2-$OCH_3$ | $CH_2OH$ | le moins polaire | base | $C_{14}H_{17}NO_4$ | 177 | $^1H$ | $CDCl_3$ | 1,6 à 2,4 (5H); 2,9 (2H) ; 3,8 (6H), 4,4 (1H), 6,6 à 6,8 (2H) ; 7,7 (1H) | 1730 |
| MD 280403 | 1 | 0 | 2-$OCH_3$ | $CH=CH_2$ | cis | base | $C_{14}H_{15}NO_3$ | 100 | $^1H$ | $CDCl_3$ | 1,7 à 2,1 (2H) ; 2,9 (2H) ; 3,8 (3H); 4,2 (1H); 4,9 à 6 (4H); 6,6 à 6,8 (2H) ; 8 (1H) | 1760 |
| MD 280404 | 1 | 0 | 2-$OCH_3$ | $CH=CH_2$ | trans | base | $C_{14}H_{15}NO_3$ | 134 | $^1H$ | $CDCl_3$ | 1,4 à 2,4 (2H); 2,8 (2H) ; 3,7 (4H); 4,5 (1H); 5,2 à 6,2 (3H); 6,6 à 6,8 (2H) ; 8,1 (1H) | 1750 |
| MD 280386 | 1 | 0 | 2-$OCH_3$ | $CH_2OH$ | cis | base | $C_{13}H_{15}NO_4$ | 162 | $^1H$ | $DMSOd_6$ | 2,1 (2H); 2,9 (2H); 3,8 à 4,8 (8H); 6,6 à 6,8 (2H) ; 8 (1H) | 1720 |

T A B L E A U   I

(suite)

| Code | n | p | $R_2$ | $R_1$ | Configuration | Base ou sel | Formule brute | F°C | R.M.N. | | | I.R.νC = 0 $cm^{-1}$ |
|---|---|---|---|---|---|---|---|---|---|---|---|---|
| | | | | | | | | | Noyau | Solvant | δ p.p.m. | |
| MD 280387 | 1 | 0 | 2-$OCH_3$ | $CH_2OH$ | trans | base | $C_{13}H_{15}NO_4$ | 147 | $^1H$ | $DMSOd_6$ | 2 (2H); 2,8 (2H); 3,6 à 4,4 (7H); 5,2 (1H); 6,6 à 6,9 (2H) ; 8 (1H) | 1720 |
| MD 280448 | 1 | 0 | 2-$OCH_3$ | $CH_2$-$OCH_3$ | cis | base | $C_{14}H_{17}NO_4$ | 99 | $^1H$ | $CDCl_3$ | 2 (2H); 2,8 (2H); 3,3 (3H) ; 3,6 (2H); 3,8 (3H); 4,2 (1H); 4,7 (1H) ; 6,6 à 6,8 (2H) ; 8 (1H) | 1740 |
| MD 280449 | 1 | 0 | 2-$OCH_3$ | $CH_2$-$OCH_3$ | trans | base | $C_{14}H_{17}NO_4$ | 105 | $^1H$ | $CDCl_3$ | 1,6 à 2,4 (2H); 2,9 (2H) ; 3,4 (3H); 3,6 à 4,4 (7H) ; 6,6 à 6,8 (2H) ; 8,1 (1H) | 1750 |
| MD 370024 | 1 | 0 | 2-$OCH_3$ | $CH_2$-$NH_2$ | cis | sel HCl | $C_{13}H_{17}ClN_2O_3$ + 6,2 % $H_2O$ | >250 | $^1H$ | $DMSOd_6$ | 2 (2H); 2,8 (2H); 3,2 (2H) ; 3,7 (3H) ; 4,3 (1H) ; 5 (1H); 6 à 6,8 (2H) ; 7,9 (1H) | 1760 |
| MD 370025 | 1 | 0 | 2-$OCH_3$ | $CH_2$-$NH_2$ | trans | sel HCl | $C_{13}H_{17}ClN_2O_3$ + 4,8 % $H_2O$ | >250 | $^1H$ | $DMSOd_6$ | 1,6 à 2,2 (2H); 2,8 (2H) ; 3,3 (2H); 3,6 (3H); 4 (1H) ; 4,5 (1H); 6,6 à 6,8 (2H) ; 7,9 (1H) | 1760 |
| MD 370026 | 1 | 0 | 2-$OCH_3$ | $CH_2$-$NHCH_3$ | cis | HCl | $C_{14}H_{19}ClN_2O_3$ | >250 | $^1H$ | $DMSOd_6$ | 2 (2H); 2,6 (3H); 2,8 (3H); 3,4 (2H); 3,7 (3H); 4,4 (1H); 5,2 (1H); 6,1 à 7 (2H); 7,9 (1H) | 1750 |

TABLEAU I

(suite)

| Code | n | p | $R_2$ | $R_1$ | Configuration | Base ou sel | Formule brute | F°C | R.M.N. Noyau | R.M.N. Solvant | R.M.N. δ p.p.m. | I.R.νC = O $cm^{-1}$ |
|---|---|---|---|---|---|---|---|---|---|---|---|---|
| MD 370027 | 1 | 0 | $2\text{-}OCH_3$ | $CH_2\text{-}NHCH_3$ | trans | sel HCl | $C_{14}H_{19}ClN_2O_3$ | >250 | $^1H$ | $DMSOd_6$ | 1,6 à 2,4 (2H); 2,6 (3H) ; 2,8 (2H); 3,4 (2H); 3,7 (3H); 4 (1H); 4,7 (1H); 6,6 à 6,9 (2H) ; 7,9 (1H) | 1750 |
| MD 370028 | 1 | 0 | $2\text{-}OCH_3$ | $CH_2N\text{-}(CH_3)_2$ | cis | sel HCl | $C_{15}H_{21}ClN_2O_3$ | >250 | $^1H$ | $DMSOd_6$ | 1,6 à 2,4 (2H); 2,8 (8H) ; 3,6 (2H); 3,7 (3H); 4,4 (1H); 5,4 (1H); 6,6 à 6,9 (2H) ; 7,8 (1H) | 1740<br>1760 |
| MD 370029 | 1 | 0 | $2\text{-}OCH_3$ | $CH_2N\text{-}(CH_3)_2$ | trans | sel HCl | $C_{15}H_{21}ClN_2O_3$ + 5,12 % $H_2O$ | >250 | $^1H$ | $DMSOd_6$ | 1,5 à 2,4 (2H); 2,8 (8H) ; 3,3 à 4,1 (6H) ; 4,9 (1H) ; 6,6 à 6,9 (2H) ; 8 (1H) | 1760 |
| MD 370067 | 2 | 0 | H | $CH_2OH$ | cis | base | $C_{13}H_{15}NO_3$ | 170 | $^1H$ | $DMSOd_6$ | 1,1 à 2,2 (4H); 2,5 à 3 (2H); 3,5 à 4 (3H); 4,7 (1H); 5,1 (1H) ; 7 à 7,5 (4H) | 1730<br>1750 |
| MD 370022 | 2 | 0 | $2\text{-}OCH_3$ | $CH_2OH$ | cis | base | $C_{14}H_{17}NO_4$ | 120 | $^1H$ | $DMSOd_6$ | 1 à 2,2 (4H); 2,7 (2H); 3,7 (6H); 4,6 (1H); 5 (1H) ; 6,6 à 6,8 (2H) ; 7,2 (1H) | 1730 |

TABLEAU I

(suite)

| Code | n | p | $R_2$ | $R_1$ | Configuration | Base ou sel | Formule brute | F°C | R.M.N. | | | I.R.νC = 0 $cm^{-1}$ |
|---|---|---|---|---|---|---|---|---|---|---|---|---|
| | | | | | | | | | Noyau | Solvant | δ p.p.m. | |
| MD 370023 | 2 | 0 | 2-$OCH_3$ | $CH_2OH$ | trans | base | $C_{14}H_{17}NO_4$ | 200 | $^1H$ | DMSO | 1,2 à 2,2 (4H); 2,7 (2H) ; 3,5 (3H); 3,7 (3H); 4,2 (1H); 5 (1H); 6,6 à 6,8 (2H) ; 7,1 (1H) | 1730 |
| MD 370317 | 1 | 1 | 2-$OCH_3$ | $CH=CH_2$ | le moins polaire | base | $C_{15}H_{17}NO_3$ | 140 | $^1H$ | $CDCl_3$ | 1,2 à 2,2 (4H) ; 2,8 (2H) ; 3,4 à 3,7 (1H); 3,7 (3H) ; 4,6 à 4,9 (1H) ; 5,1 à 6,2 (3H) ; 6,5 à 6,9 (2H) ; 7,6 (1H) | 1680 |
| MD 370318 | 1 | 1 | 2-$OCH_3$ | $CH=CH_2$ | le plus polaire | base | $C_{15}H_{17}NO_3$ | 106 | $^1H$ | $CDCl_3$ | 1,8 à 2,3 (4H) ; 2,8 (2H) ; 3,4 à 3,7 (1H) ; 3,7 (3H) ; 4,6 à 6,3 (4H) ; 6,5 à 6,9 (2H) ; 7,8 (1H) | 1690 |
| MD 370017 | 2 | 0 | H | $CH=CH_2$ | cis | base | $C_{14}H_{15}NO_2$ | 131 | $^1H$ | $CDCl_3$ | 1,2 à 2,3 (4H); 2,7 (2H) ; 3,4 à 3,9 (1H) ; 5 à 6,3 (4H) ; 7 à 7,5 (4H) | 1750 |
| MD 370018 | 2 | 0 | H | $CH=CH_2$ | trans | base | $C_{14}H_{15}NO_2$ | 95 | $^1H$ | $CDCl_3$ | 1 à 2,3 (4H) ; 2,6 à 2,9 (2H); 3,1 à 3,5 (1H); 4,6 (1H); 5,1 à 6,3 (3H) ; 7 à 7,6 (4H) | 1750 |

TABLEAU I

(suite)

| Code | n | p | $R_2$ | $R_1$ | Confi-guration | Base ou sel | Formule brute | F°C | R.M.N. | | | I.R.νC = 0 $cm^{-1}$ |
|---|---|---|---|---|---|---|---|---|---|---|---|---|
| | | | | | | | | | Noyau | Solvant | δ p.p.m. | |
| MD 370333 | 1 | 0 | 3-Cl | $CH=CH_2$ | trans | base | $C_{13}H_{12}ClNO_2$ | huile | $^1H$ | $CDCl_3$ | 1,2 à 2,2 (2H); 2,6 à 3 (2H); 3,9 à 4,4 (1H); 5,9 à 6,2 (4H); 6,8 à 7,2 (2H) ; 8,2 (1H) | 1750 |
| MD 370334 | 1 | 0 | 3-Cl | $CH=CH_2$ | cis | base | $C_{13}H_{12}ClNO_2$ | huile | $^1H$ | $CDCl_3$ | 1 à 2,5 (2H); 2,7 à 3,1 (2H); 3,5 à 4 (1H); 4,5 à 4,7 (1H); 5,3 à 6,3 (3H) ; 7 (3H) ; 8,4 (1H) | 1750 |
| MD 370240 | 1 | 0 | $3-CH_3$ | $CH=CH_2$ | cis | base | $C_{14}H_{15}NO_2$ | huile | $^1H$ | $CDCl_3$ | 1,2 à 2,2 (2H); 2,3 (3H) ; 2,7 à 3,1 (2H); 3,8 à 4,4 (1H); 4,9 à 6,3 (4H); 6,7 à 7,2 (2H) ; 8,0 (1H) | - |
| MD 370239 | 1 | 0 | $3-CH_3$ | $CH=CH_2$ | trans | base | $C_{14}H_{15}NO_2$ | huile | $^1H$ | $CDCl_3$ | 1,2 à 2,4 (2H); 2,4 (3H) ; 2,7 à 3,1 (2H); 3,5 à 4 (1H); 4,6 (1H); 5,3 à 6,3 (3H) ; 6,7 à 7,2 (2H) ; 8,1 (1H) | - |

TABLEAU I

(suite)

| Code | n | p | $R_2$ | $R_1$ | Configuration | Base ou sel | Formule brute | F°C | R.M.N. Noyau | R.M.N. Solvant | R.M.N. δ p.p.m. | I.R. $\nu C=O$ $cm^{-1}$ |
|---|---|---|---|---|---|---|---|---|---|---|---|---|
| MD 370250 | 2 | 0 | 2-$OCH_3$ | $CH=CH_2$ | cis | base | $C_{15}H_{17}NO_3$ | 175 | $^1H$ | $CDCl_3$ | 1 à 2,3 (4H); 2,5 à 2,9 (2H); 3,4 à 3,8 (1H); 3,8 (3H) ; 5 à 6,3 (4H) ; 6,6 à 6,9 (2H) ; 7,2 (1H) | 1750 |
| MD 370251 | 2 | 0 | 2-$OCH_3$ | $CH=CH_2$ | trans | base | $C_{15}H_{17}NO_3$ | 123 | $^1H$ | $CDCl_3$ | 1,1 à 2,3 (4H)) ; 2,6 à 2,9 (2H) ; 3,1 à 3,5 (1H) ; 3,7 (3H) ; 4,5 (1H) ; 5,1 à 6,3 (3H) ; 6,6 à 6,9 (2H); 7,3 (1H) | 1750 |

Les composés de formule (I) et leurs sels physiologiquement acceptables ont été testés in vitro et in vivo et se sont mentrés doués d'une activité thérapeutique intéressante. Ces composés et sels se sont en particulier révélés actifs en tant qu'inhibiteurs de la monoamine oxydase et capables de potentialiser les effets de la sérotonine lorsqu'elle est administrée chez l'animal sous forme du précurseur hydroxy-5 tryptophane

Cette activité a été mise en évidence :

- in vitro sur des hemogénats de cerveau de rat, en mesurant la concentration de produit inhibant de 50 % ($CI_{50}$) l'activité de la monoamine oxydase en utilisant la sérotonine comme substrat à 480 $\mu$M, d'après la méthode décrite par M. STROLIN BENEDETTI, T. BOUCHER et C.J. FOWLER dans Naunyn - Schmiedebergs Archiv. of Pharm. (1983) 323, 315-320 ;
- in vivo, en déterminant chez le rat la dose de produit qui, administrée par voie orale, provoque chez 50 % des animaux ($DE_{50}$) l'apparition de tremblements généralisés ou de stéréotypies (pianotage, mouvements de tête) censécutive à l'administration par voie intrapéritonéale 1 h après le premier traitement, d'une dose de 120 mg/kg d'hydroxy-5 tryptophane (5-HTP) [M. JALFRE, B. BUCHER, A. COSTON, G. MOCQUET, R.D. PORSOLT, Arch. Int. Pharmacodyn., (1982), 259, 194-221].

A titre d'exemple, l'activité de quelques composés selon l'invention est rassemblée dans le tableau II suivant :

TABLEAU II

| Composé testé No. de code | Test in vivo | Test in vitro |
|---|---|---|
| | $DE_{50}$ (mg/kg/p.o.) | $CI_{50}$ ($\mu$M) |
| MD 280430 | 105 | 1,4 |
| MD 200682 | 2,3 | 0,09 |
| MD 280404 | 11 | 0,029 |
| MD 200602 | | 2,8 |
| MD 200718 | | 8,7 |
| MD 200502 | | 0,5 |
| MD 200503 | 20 | 0,057 |
| MD 200504 | 7,8 | |
| MD 280448 | 1,8 | 0,009 |
| MD 280449 | | 0,006 |
| MD 370024 | | 1 |
| MD 370025 | | 1,6 |
| MD 370027 | 5,8 | |
| MD 280386 | | 0,019 |
| MD 280387 | | 0,075 |
| MD 370336 | | 0,08 |
| MD 370335 | | 0,097 |

Par ailleurs, il n'a pas été constaté de signes toxiques particuliers chez le rongeur par administration des composés selon l'invention aux doses auxquelles ils font preuve d'une activité pharmacologique. Ainsi par exemple, les composés MD 280 449 et MD 370 026 possèdent une $DL_{50}$ chez la souris respectivement de 1120 et 263 mg/kg/p.o..

Les données ci-dessus montrent l'intérêt thérapeutique des composés de formule (I) et de leurs sels physiologiquement acceptables selon l'invention. Ces composés et sels trouvent donc leur application en tant que médicaments pour l'homme et l'animal, en particulier peur le traitement des états dépressifs.

La présente invention s'étend par ailleurs à des compositions pharmaceutiques ou vétérinaires contenant au moins l'un desdits composés et sels ainsi qu'un ou plusieurs véhicules physiologiquement acceptables. Ces compositions peuvent être formulées notamment en vue de leur administration orale et se présenter alors par exemple sous forme de dragées, gélules, comprimés ou solutions buvables, ou en vue de leur administration parentérale et se présenter alors sous la forme de solutions injectables.

Enfin, les doses auxquelles les composés selon l'invention peuvent être administrés dépendra notamment de la voie d'administration, du poids corporel du patient, de l'état de ce dernier et de la puissance thérapeutique des composés mis en oeuvre. Généralement, les doses pourront atteindre 1 à 50 mg/kg/jour, en une ou plusieurs prises.

**Revendications**

**Revendications pour les Etats contractants suivants : AT, DE, GB, FR, IT, NL, SE, LI, CH, BE, LU**

**1.** Carbamate tricyclique répondant à la formule

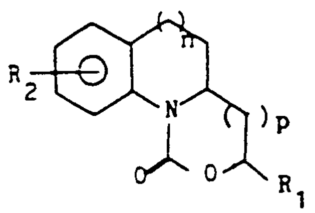

(I)

dans laquelle :

- n = 0, 1 ou 2,
- p = 0 ou 1,
- $R_1$ représente un atome d'hydrogène ; un groupe alkyle en $C_1$-$C_4$ ; un groupe phényle ; un groupe alkényle en $C_2$-$C_3$ ; ou un groupe méthyle substitué par un groupe hydroxy, un groupe alkoxy en $C_1$-$C_4$, un groupe amino ou un groupe N-alkylamino ou N,N-dialkylamino où le reste alkyle est en $C_1$-$C_4$, et
- $R_2$ représente un atome d'hydrogène ; un atome d'halogène ; un groupe alkyle en $C_1$-$C_4$ ; ou un groupe alkoxy en $C_1$-$C_4$ ;

ainsi que les sels d'addition d'acide minéral ou organique, du carbamate de formule (I) comportant un groupe salifiable par un tel acide.

**2.** carbamate tricyclique ou sel selon la revendication 1, par utilisation en tant que médicament à usage humain ou vétérinaire.

**3.** Composition pharmaceutique ou vétérinaire comprenant (a) en tant que principe actif un carbamate tricyclique ou sel selon la revendication 1 et (b) un véhicule, physiologiquement acceptable, pour ce carbamate et sel.

**4.** Utilisation d'un carbamate tricyclique ou sel selon la revendications 1, pour la préparation d'un agent antidépresseur.

**5.** Procédé de préparation du carbamate tricyclique et des sels selon la revendication 1, caractérisé en ce qu'il comprend

(i) la cyclisation, par le carbonate d'éthyle et en présence d'une base ou par le phosgène, du composé de formule

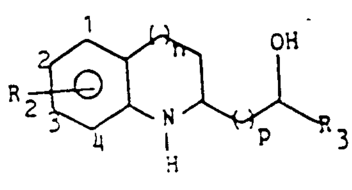

(II)

dans laquelle n, p et $R_2$ ont la même signification que dans la formule (I) définie à la revendication 1 et $R_3$ représente un atome d'hydrogène, un groupe alkyle en $C_1$-$C_4$, un groupe alkényle en $C_2$-$C_3$ ou un noyau phényle, ou

la cyclisation intramoléculaire en présence d'une base du composé de formule

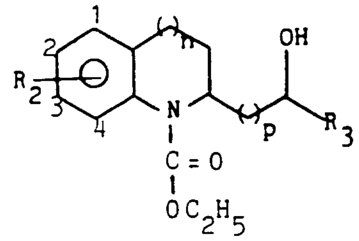

(III)

dans laquelle n, p, $R_2$ et $R_3$ ont la même signification que dans la formule (II) ci-dessus, ce qui conduit au composé de formule (Ia) et de structure particulière

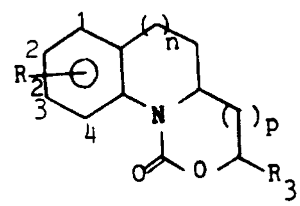

(Ia)

dans laquelle n, p, $R_2$ et $R_3$ ont la même signification que dans la formule (II),
(ii) éventuellement l'ozonolyse du composé de formule (Ia) pour lesquels $R_3$ représente un groupe $CH=CH_2$, cette ozonolyse étant suivie d'une réduction de préférence par un hydrure métallique, ce qui conduit au composé de formule (Ib) et de structure particulière

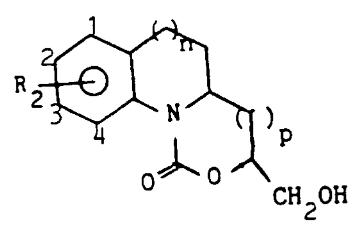

(Ib)

où n, p et $R_2$ ont la même signification que dans la formule (Ia),
(iii) éventuellement l'action d'un agent d'alkylation, en présence d'un catalyseur de transfert de phase, sur le reste $CH_2OH$ du composé de formule (Ib) pour obtenir le composé de formule (Ic) et de structure particulière

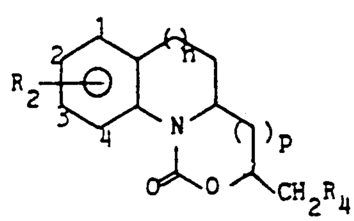

(Ic)

où n, p et $R_2$ ont la même signification que dans la formule (Ib) et $R_4$ représente un groupe alkoxy en $C_1$-$C_4$,
(iv) éventuellement l'action d'un halogénure de mésyle ou de tosyle sur le composé de formule (Ib), suivie de l'action soit du borohydrure de sodium dans le diméthylsulfoxyde, soit de $NH_3$ ou d'une mono- ou dialkylamine dont le ou les restes alkyle sont en $C_1$-$C_4$, sur le mésylate ou tosylate résultant, ce qui conduit au composé de formule (Id) et de structure particulière

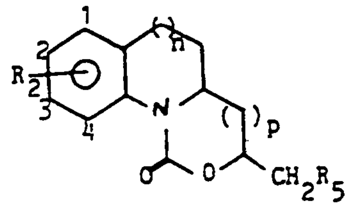

(Id)

où n, p et $R_2$ ont la même signification que dans la formule (Ib) et $R_5$ représente un atome d'hydrogène, un groupe amino ou un groupe alkylamino ou dialkylamino dont le ou les restes alkyle sont en $C_1$-$C_4$, et

(v) éventuellement la salification par un acide minéral ou organique du composé de formule (Id).

**Revendications pour les Etats contractants suivants : ES, GR**

**1.** Procédé de préparation du carbamate tricyclique répondant à la formule

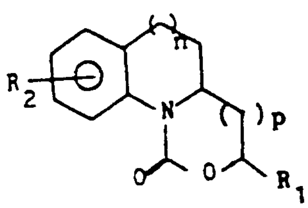

(I)

dans laquelle :

- n = 0, 1 ou 2,
- p = 0 ou 1,
- $R_1$ représente un atome d'hydrogène ; un groupe alkyle en $C_1$-$C_4$ ; un groupe phényle ; un groupe alkényle en $C_2$-$C_3$ ; ou un groupe méthyle substitué par un groupe hydroxy, un groupe alkoxy en $C_1$-$C_4$, un groupe amino ou un groupe N-alkylamino ou N,N-dialkylamino où le reste alkyle est en $C_1$-$C_4$, et
- $R_2$ représente un atome d'hydrogène ; un atome d'halogène ; un groupe alkyle en $C_1$-$C_4$ ; ou un groupe alkoxy en $C_1$-$C_4$ ;

ainsi que des sels d'addition d'acide minéral ou organique, du carbamate de formule (I) comportant un groupe salifiable par un tel acide,

caractérisé en ce qu'il comprend

(i) la cyclisation, par le carbonate d'éthyle et en présence d'une base ou par le phosgène, du composé de formule

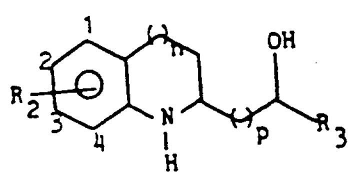

(II)

dans laquelle n, p et $R_2$ ont la même signification que dans la formule (I) définie ci-dessus et $R_3$ représente un atome d'hydrogène, un groupe alkyle en $C_1$-$C_4$, un groupe alkényle en $C_2$-$C_3$ ou un noyau phényle, ou

la cyclisation intramoléculaire en présence d'une base du composé de formule

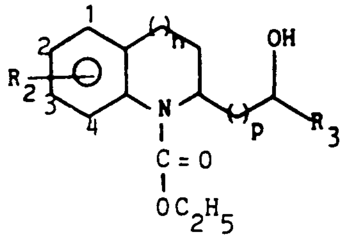

(III)

dans laquelle n, p, $R_2$ et $R_3$ ont la même signification que dans la formule (II) ci-dessus, ce qui conduit au composé de formule (Ia) et de structure particulière

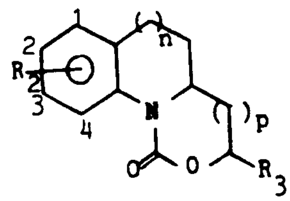

(Ia)

dans laquelle n, p, $R_2$ et $R_3$ ont la même signification que dans la formule (II),
(ii) éventuellement l'ozonolyse du composé de formule (Ia) pour lesquels $R_3$ représente un groupe $CH=CH_2$, cette ozonolyse étant suivie d'une réduction de préférence par un hydrure métallique, ce qui conduit au composé de formule (Ib) et de structure particulière

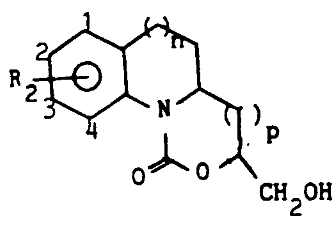

(Ib)

où n, p et $R_2$ ont la même signification que dans la formule (Ia),
(iii) éventuellement l'action d'un agent d'alkylation, en présence d'un catalyseur de transfert de phase, sur le reste $CH_2OH$ du composé de formule (Ib) pour obtenir le composé de formule (Ic) et de structure particulière

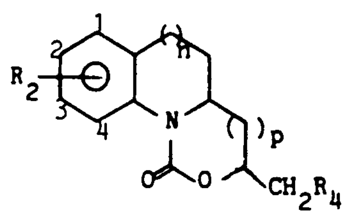

(Ic)

où n, p et $R_2$ ont la même signification que dans la formule (Ib) et $R_4$ représente un groupe alkoxy en $C_1$-$C_4$,
(iv) éventuellement l'action d'un halogénure de mésyle ou de tosyle sur le composé de formule (Ib), suivie de l'action soit du borohydrure de sodium dans le diméthylsulfoxyde, soit de $NH_3$ ou d'une mono- ou dialkylamine dont le ou les restes alkyle sont en $C_1$-$C_4$, sur le mésylate ou tosylate résultant, ce qui conduit au composé de formule (Id) et de structure particulière

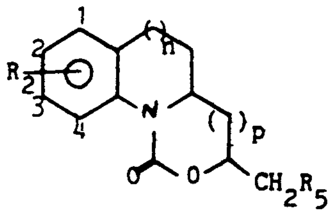

(Id)

où n, p et $R_2$ ont la même signification que dans la formule (Ib) et $R_5$ représente un atome d'hydrogène, un groupe amino ou un groupe alkylamino ou dialkylamino dont le ou les restes alkyle sont en $C_1$-$C_4$, et

(v) éventuellement la salification par un acide minéral ou organique du composé de formule (Id).

**2.** Procédé selon la revendication 1, caractérisé en ce que la base mise en oeuvre à l'étape (i) est un alcoolate de métal alcalin.

**3.** Procédé selon la revendication 1 ou 2, caractérisé en ce que l'agent d'alkylation et le catalyseur de transfert de phase mis en oeuvre à l'étape (iii) sont respectivement un sulfate de dialkyle en $C_1$-$C_4$ et le bromure de tétrabutylammonium.

**4.** Procédé de préparation d'une composition pharmaceutique ou vétérinaire pour le traitement des états dépressifs, caractérisé en ce qu'il consiste à mélanger un carbamate tricyclique de formule (I) définie à la revendication 1 ou un sel d'addition d'acide minéral ou organique de ce carbamate, avec un véhicule, physiologiquement acceptable, pour ce carbamate et sel.

**5.** Utilisation d'un carbamate tricyclique de formule (I) définie à la revendication 1 ou d'un sel d'addition d'acide minéral ou organique de ce carbamate, pour la préparation d'un agent antidépresseur.

**Claims**

**Claims for the following Contracting States : AT, DE, GB, FR, IT, NL, SE, LI, CH, BE, LU**

**1.** Tricyclic carbamate complying with the formula

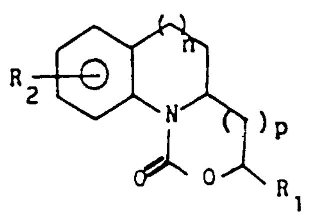

(I)

in which:

- n = 0, 1 or 2,
- p = 0 or 1,
- $R_1$ represents a hydrogen atom; a group which is alkyl in $C_1$-$C_4$; a phenyl group; a group which is alkenyl in $C_2$-$C_3$; or a methyl group substituted by a hydroxy group, a group which is alkoxy in $C_1$-$C_4$, an amino group or a N-alkylamino or N,N-dialkylamino group where the alkyl radical is in $C_1$-$C_4$, and
- $R_2$ represents a hydrogen atom; a halogen atom; a group which is alkyl in $C_1$-$C_4$; or a group which is alkoxy in $C_1$-$C_4$;

as well as the salts of addition of mineral or organic acid of the carbamate of formula (I) having a group which is salifiable by such an acid.

**2.** Tricyclic carbamate or salt as claimed in claim 1, for use as a medicament for human or veterinary use.

**3.** Pharmaceutical or veterinary composition including

(a) a tricyclic carbamate or salt as claimed in claim 1 as active principle and (b) a physiologically acceptable vehicle for this carbamate and salt.

**4.** Use of a tricyclic carbamate or salt as claimed in claim 1 for the preparation of an antidepressant agent.

**5.** Process of preparation of the tricyclic carbamate and salts as claimed in claim 1, characterised in that it includes

(i) the cyclisation by ethyl carbonate and in the presence of a base or by phosgene of the compound of the formula

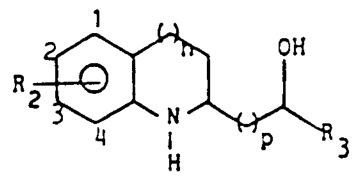

(II)

in which n, p and $R_2$ have the same significance as in formula (I) defined in claim 1 and $R_3$ represents a hydrogen atom, a group which is alkyl in $C_1$-$C_4$, a group which is alkenyl in $C_2$-$C_3$ or a phenyl nucleus, or

the intramolecular cyclisation in the presence of a base of the compound of formula

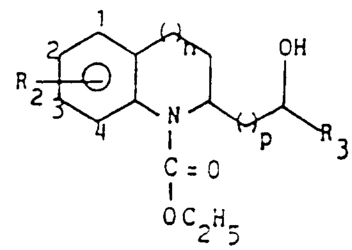

(III)

in which n, p, $R_2$ and $R_3$ have the same significance as in formula (II) above, which leads to the compound of formula (Ia) with the particular structure

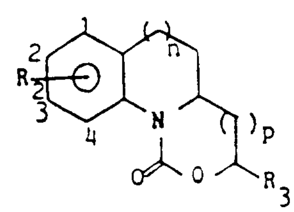

(Ia)

in which n, p, $R_2$ and $R_3$ have the same significance as in formula (II),

(ii) possibly the ozonolysis of the compound of formula (Ia) for which $R_3$ represents a $CH = CH_2$ group, this ozonolysis being followed by a reduction preferably by a metal hydride, which leads to the compound of formula (Ib) with the particular structure

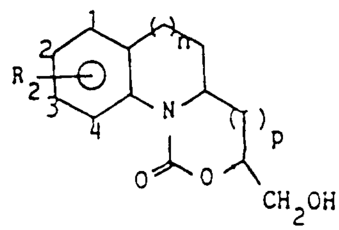

(Ib)

where n, p and $R_2$ have the same significance as in formula (Ia),

(iii) possibly the action of an alkylation agent, in the presence of a phase transfer catalyst, on the $CH_2OH$ radical of the compound of formula (Ib) in order to obtain the compound of formula (Ic) with the particular structure

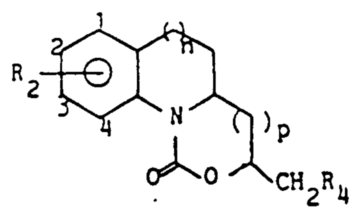

(Ic)

where n, p and $R_2$ have the same significance as in formula (Ib) and $R_4$ represents a group which is alkoxy in $C_1$-$C_4$,

(iv) possibly the action of a mesyl or tosyl halide on the compound of formula (Ib), followed by the action either of sodium borohydride in dimethylsulphoxide or of $NH_3$ or of a mono- or dialkylamine of which the alkyl radical or radicals are in $C_1$-$C_4$, on the resulting mesylate or tosylate, which leads to the compound of formula (Id) with the particular structure

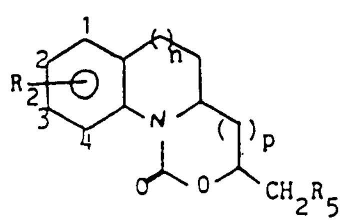

(Id)

where n, p and $R_2$ have the same significance as in formula (Ib) and $R_5$ represents a hydrogen atom, an amino group or an alkylamino or dialkylamino group of which the alkyl radical or radicals are in $C_1$-$C_4$, and

(v) possibly the salification by a mineral or organic acid of the compound of formula (Id).

**Claims for the following Contracting States : ES, GR**

**1.** Process of preparation of the tricyclic carbamate complying with the formula :

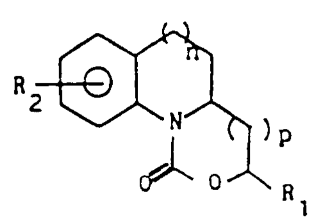

(I)

in which:

- n = 0, 1 or 2,
- p = 0 or 1,
- $R_1$ represents a hydrogen atom; a group which is alkyl in $C_1$-$C_4$; a phenyl group; a group which is alkenyl in $C_2$-$C_3$; or a methyl group substituted by a hydroxy group, a group which is alkoxy in $C_1$-$C_4$, an amino group or a N-alkylamino or N,N-dialkylamino group where the alkyl radical is in $C_1$-$C_4$, and
- $R_2$ represents a hydrogen atom; a halogen atom; a group which is alkyl in $C_1$-$C_4$; or a group which is alkoxy in $C_1$-$C_4$;

as well as ofthe salts of addition of mineral or organic acid of the carbamate of formula (I) having a group which is salifiable by such an acid,
characterized in that it includes

(i) the cyclisation by ethyl carbonate and in the presence of a base or by phosgene of the compound of the formula

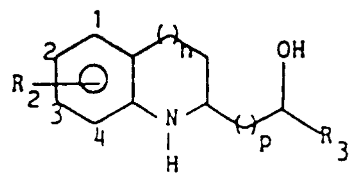

(II)

in which n, p and $R_2$ have the same significance as in formula (I) defined in claim 1 and $R_3$ represents a hydrogen atom, a group which is alkyl in $C_1$-$C_4$, a group which is alkenyl in $C_2$-$C_3$ or a phenyl nucleus, or

the intramolecular cyclisation in the presence of a base of the compound of formula

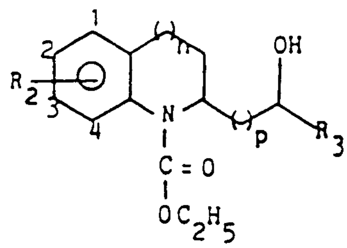

(III)

in which n, p, $R_2$ and $R_3$ have the same significance as in formula (II) above ,
which leads to the compound of formula (Ia) with the particular structure

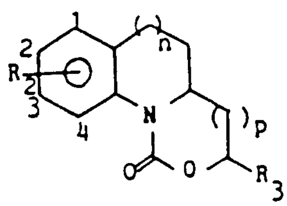

(Ia)

in which n, p, $R_2$ and $R_3$ have the same significance as in formula (II) ,
(ii) possibly the ozonolysis of the compound of formula (Ia) for which $R_3$ represents a $CH=CH_2$ group, this ozonolysis being followed by a reduction preferably by a metal hydride, which leads to the compound of formula (Ib) with the particular structure

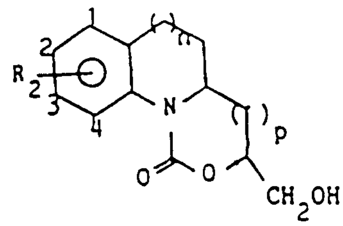

(Ib)

where n, p and $R_2$ have the same significance as in formula (Ia) ,

(iii) possibly the action of an alkylation agent, in the presence of a phase transfer catalyst, on the $CH_2OH$ radical of the compound of formula (Ib) in order to obtain the compound of formula (Ic) with the particular structure

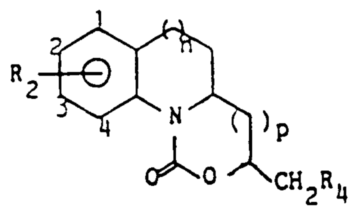

(Ic)

where n, p and $R_2$ have the same significance as in formula (Ib) and $R_4$ represents a group which is alkoxy in $C_1$-$C_4$ ,

(iv) possibly the action of a mesyl or tosyl halide on the compound of formula (Ib), followed by the action either of sodium borohydride in dimethylsulphoxide or of $NH_3$ or of a mono- or dialkylamine of which the alkyl radical or radicals are in $C_1$-$C_4$, on the resulting mesylate or tosylate, which leads to the compound of formula (Id) with the particular structure

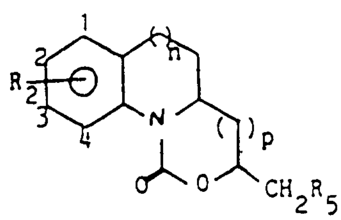

(Id)

where n, p and $R_2$ have the same significance as in formula (Ib)and $R_5$ represents a hydrogen atom, on amino group or an alkylamino or dialkylamino group of which the alkyl radical or radicals are in $C_1$-$C_4$, and

(v) possibly the salification by a mineral or organic acid of the compound of formula (Id).

**2.** Process according to claim 1, characterized in that the base used in step (i) is an alkali metal alcoholate.

**3.** Process according to claim 1 or 2, characterized in that the alkylation agent and phase transfer catalyst used in step (iii) are a di($C_1$-$C_4$ alkyl) sulphate and the tetrabutylammonium bromide, respectively.

**4.** Process of preparation of a pharmaceutical or veterinary composition for the treatment of depressive conditions, characterized in that it consists in mixing a tricyclic carbamate of formula (I) defined in claim 1 or a salt of addition of mineral or organic acid of said carbamate, with a physiologically acceptable vehicle for this carbamate or salt.

**5.** Use of a tricyclic carbamate of formula (I) defined in claim 1 or of a salt of addition of mineral or organic acid of said carbamate, for the preparation of an antidepressant agent.

**Patentansprüche**
**Patentansprüche für folgende Vertragsstaaten : AT, DE, GB, FR, IT, NL, SE, LI, CH, BE, LU**

1. Tricyclisches Carbamat mit der Formel

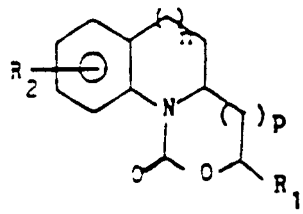

(I)

in welcher:
n = 0,1 oder 2,
p = 0 oder 1,
$R_1$ ein Wasserstoffatom; eine $C_1$-$C_4$-Alkylgruppe; eine Phenylgruppe; eine $C_2$-$C_3$-Alkenylgruppe; oder eine Methylgruppe, die durch eine Hydroxygruppe, eine $C_1$-$C_4$-Alkoxygruppe, eine Aminogruppe oder eine N-Alkylaminogruppe oder N,N-Dialkylaminogruppe, deren Alkylrest $C_1$-$C_4$ ist, substituiert ist, bedeutet, und
$R_2$ ein Wasserstoffatom; ein Halogenatom; eine $C_1$-$C_4$-Alkylgruppe; oder eine $C_1$-$C_4$-Alkoxygruppe bedeutet;
sowie die Additionssalze, mit einer anorganischen oder organischen Säure, des Carbamats der Formel (I), welches eine durch eine solche Säure in das Salz überführbare Gruppe enthält.

2. Tricyclisches Carbamat oder Salz nach Anspruch 1, zur Verwendung als Medikament zur Verwendung beim Menschen oder in der Tiermedizin.

3. Pharmazeutische oder tierärztliche Zusammensetzung umfassend (a) als wirksamen Bestandteil ein tricyclisches Carbamat oder Salz nach Anspruch 1 und (b) einen physiologisch annehmbaren Träger für dieses Carbamat und Salz.

4. Verwendung eines tricyclischen Carbamats oder Salzes nach Anspruch 1 zur Herstellung eines antidepressiven Mittels.

5. Verfahren zur Herstellung des tricyclischen Carbamats und der Salze nach Anspruch 1, **dadurch gekennzeichnet**, daß es umfaßt
(i) die Cyclisierung, durch Ethylcarbonat und in Gegenwart einer Base oder durch Phosgen, der Verbindung der Formel

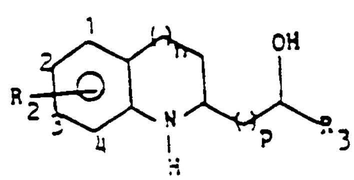

(II)

in welcher n, p und $R_2$ die gleiche Bedeutung wie in Formel (I) haben, die in Anspruch 1 definiert ist, und $R_3$ ein Wasserstoffatom, eine $C_1$-$C_4$-Alkylguppe, eine $C_2$-$C_3$-Alkenylgruppe oder einen Phenylring bedeutet, oder
die intramolekulare Cyclisierung der Verbindung der Formel

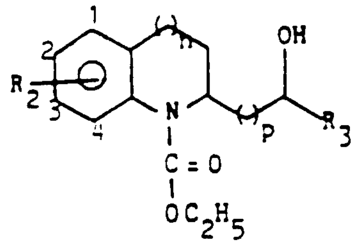

(III)

in welcher n, p, $R_2$ und $R_3$ die gleiche Bedeutung wie in der Formel (II) oben haben, in Gegenwart einer Base,
was zu der Verbindung der Formel (Ia) und der speziellen Struktur

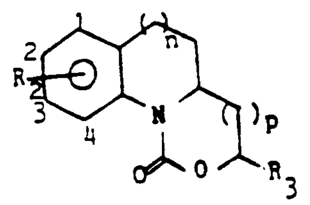

(Ia)

führt, in welcher n, p, $R_2$ und $R_3$ die gleiche Bedeutung wie in Formel (II) haben,
(ii) eventuell die Ozonolyse der Verbindung der Formel (Ia), für die $R_3$ eine $CH=CH_2$-Gruppe bedeutet, wobei auf diese Ozonolyse eine Reduktion vorzugsweise durch ein Metallhydrid folgt, was zu der Verbindung der Formel (Ib) und der speziellen Struktur

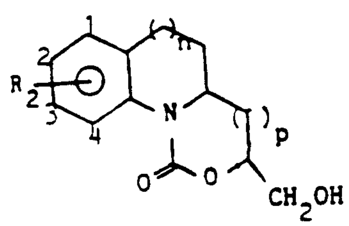

(Ib)

führt, worin n, p und $R_2$ die gleiche Bedeutung wie in Formel (Ia) haben,
(iii) eventuell die Einwirkung eines Alkylierungsmittels in Gegenwart eines Phasentransferkatalysators auf den $CH_2OH$-Rest der Verbindung der Formel (Ib), um die Verbindung der Formel (Ic) und der speziellen Struktur

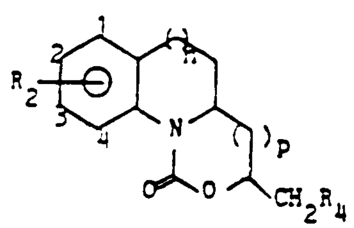

(Ic)

zu erhalten, worin n, p und $R_2$ die gleiche Bedeutung wie in der Formel (Ib) haben und $R_4$ eine $C_1$-$C_4$-Alkoxygruppe bedeutet,
(iv) eventuell die Einwirkung eines Mesyl- oder Tosylchlorids auf die Verbindung der Formel (Ib), gefolgt von der Einwirkung von entweder Natriumborhydrid in Dimethylsulfoxid oder von $NH_3$ oder einem Mono- oder Dialkylamin, dessen Alkylrest oder Alkylreste $C_1$-$C_4$ sind, auf das sich ergebende Mesylat oder Tosylat, was zu der Verbindung der Formel (Id) und der speziellen Struktur

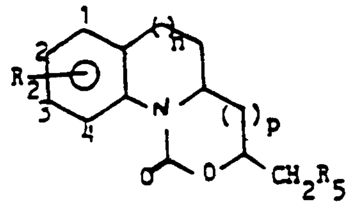

(Id)

führt, worin n, p und $R_2$ die gleiche Bedeutung wie in Formel (Ib) haben und $R_5$ ein Wasserstoffatom, eine Aminogruppe oder eine Alkylaminogruppe oder Dialkylaminogruppe, deren Alkylrest oder Alkylreste $C_1$-$C_4$ sind, bedeutet, und
(v) eventuell die Bildung eines Salzes der Verbindung der Formel (Id) mit einer anorganischen oder organischen Säure.

**Patentansprüche für folgende Vertragsstaaten : ES, GR**

**1.** Verfahren zur Herstellung des tricyclischen Carbamats mit der Formel

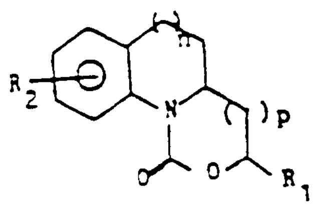

(I)

in welcher:
n = 0,1 oder 2,
p = 0 oder 1,
$R_1$ ein Wasserstoffatom; eine $C_1$-$C_4$-Alkylgruppe; eine Phenylgruppe; eine $C_2$-$C_3$-Alkenylgruppe; oder eine Methylgruppe, die durch eine Hydroxygruppe, eine $C_1$-$C_4$-Alkoxygruppe, eine Aminogruppe oder eine N-Alkylaminogruppe oder N,N-Dialkylaminogruppe, deren Alkylrest $C_1$-$C_4$ ist, substituiert ist, bedeutet, und
$R_2$ ein Wasserstoffatom; ein Halogenatom; eine $C_1$-$C_4$-Alkylgruppe; oder eine $C_1$-$C_4$-Alkoxygruppe bedeutet;
sowie der Additionssalze, mit einer anorganischen oder organischen Säure, des Carbamats der Formel (I), welches eine durch eine solche Säure in das Salz überführbare Gruppe enthält,
**dadurch gekennzeichnet**, daß es umfaßt
(i) die Cyclisierung, durch Ethylcarbonat und in Gegenwart einer Base oder durch Phosgen, der Verbindung der Formel

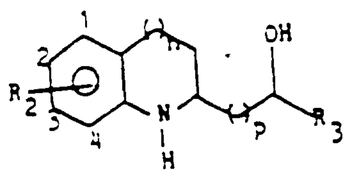

(II)

in welcher n, p und $R_2$ die gleiche Bedeutung wie in Formel (I) haben, die weiter oben definiert ist, und $R_3$ ein Wasserstoffatom, eine $C_1$-$C_4$-Alkylguppe, eine $C_2$-$C_3$-Alkenylgruppe oder einen Phenylring bedeutet, oder
die intramolekulare Cyclisierung der Verbindung der Formel

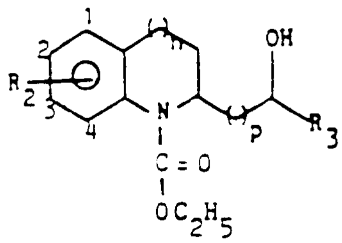

(III)

in welcher n, p, $R_2$ und $R_3$ die gleiche Bedeutung wie in der Formel (II) oben haben, in Gegenwart einer Base,
was zu der Verbindung der Formel (Ia) und der speziellen Struktur

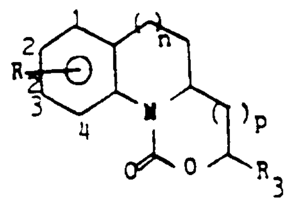

(Ia)

führt, in welcher n, p, $R_2$ und $R_3$ die gleiche Bedeutung wie in Formel (II) haben,
(ii) eventuell die Ozonolyse der Verbindung der Formel (Ia), für die $R_3$ eine $CH=CH_2$-Gruppe bedeutet, wobei auf diese Ozonolyse eine Reduktion vorzugsweise durch ein Metallhydrid folgt, was zu der Verbindung der Formel (Ib) und der speziellen Struktur

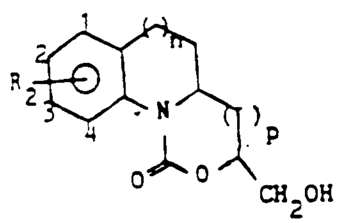

(Ib)

führt, worin n, p und $R_2$ die gleiche Bedeutung wie in Formel (Ia) haben,
(iii) eventuell die Einwirkung eines Alkylierungsmittels in Gegenwart eines Phasentransferkatalysators auf den $CH_2OH$-Rest der Verbindung der Formel (Ib), um die Verbindung der Formel (Ic) und der speziellen Struktur

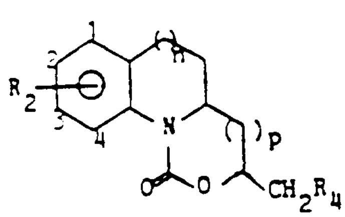

(Ic)

zu erhalten, worin n, p und $R_2$ die gleiche Bedeutung wie in der Formel (Ib) haben und $R_4$ eine $C_1$-$C_4$-Alkoxygruppe bedeutet,
(iv) eventuell die Einwirkung eines Mesyl- oder Tosylchlorids auf die Verbindung der Formel (Ib), gefolgt von der Einwirkung von entweder Natriumborhydrid in Dimethylsulfoxid oder von $NH_3$ oder einem Mono- oder Dialkylamin, dessen Alkylrest oder Alkylreste $C_1$-$C_4$ sind, auf das sich ergebende Mesylat oder Tosylat, was zu der Verbindung der Formel (Id) und der speziellen Struktur

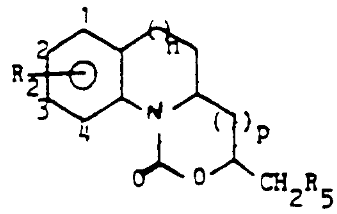

(Id)

führt, worin n, p und $R_2$ die gleiche Bedeutung wie in Formel (Ib) haben und $R_5$ ein Wasserstoffatom, eine Aminogruppe oder eine Alkylaminogruppe oder Dialkylaminogruppe, deren Alkylrest oder Alkylreste $C_1$-$C_4$ sind, bedeutet, und
(v) eventuell die Bildung eines Salzes der Verbindung der Formel (Id) mit einer anorganischen oder organischen Säure.

2. Verfahren nach Anspruch 1, **dadurch gekennzeichnet**, daß die im Schritt (i) eingesetzte Base ein Alkalimetallalkoholat ist.

3. Verfahren nach Anspruch 1 oder 2, **dadurch gekennzeichnet**, daß das Alkylierungsmittel und der Phasentransferkatalysator, die im Schritt (iii) eingesetzt werden, ein $C_1$-$C_4$-Dialkylsulfat bzw. Tetrabutylammoniumbromid sind.

4. Verfahren zur Herstellung einer pharmazeutischen oder tierärztlichen Zusammensetzung zur Behandlung von depressiven Zuständen, **dadurch gekennzeichnet**, daß es darin besteht, ein tricyclisches Carbamat der Formel (I), die im Anspruch 1 definiert ist, oder ein Additionssalz dieses Carbamats mit einer anorganischen oder organischen Säure mit einem physiologisch annehmbaren Träger für dieses Carbamat und Salz zu vermischen.

5. Verwendung eines tricyclischen Carbamats der Formel (I), die im Anspruch 1 definiert ist, oder eines Additionssalzes dieses Carbamats mit einer anorganischen oder organischen Säure zur Herstellung eines antidepressiven Mittels.